# EUROPEAN PATENT SPECIFICATION

(11) **EP 2 647 995 B1**
(45) Date of publication and mention of the grant of the patent: **10.12.2014**
(21) Application number: 12162884.6
(22) Date of filing: 02.04.2012
(51) Int. Cl.: G01N 33/68

(54) **Methods and use for assessing chronic obstructive pulmonary disease (COPD) severity**
Verfahren und Anwendung zur Beurteilung der Schwere einer chronisch obstruktiven Lungenerkrankung (COPD)
Procédés et utilisations pour évaluer la gravité d'une maladie pulmonaire obstructive chronique (COPD)

(43) Date of publication of application: 09.10.2013
(73) Proprietor: Roche Diagnostics GmbH, 68305 Mannheim (DE)
(72) Inventor: Horsch, Andrea, 68259 Mannheim (DE); Gallusser, Andreas, 82377 Penzberg (DE); Hess, Georg, 55130 Mainz (DE); Klemt, Volker, 82362 Weilheim (DE); Zdunek, Dietmar, 82327 Tutzing (DE)
(74) Representative: Schiweck, Weinzierl & Koch

(56) References cited:
- WO-A2-2006/097244
- GRIESE MATTHIAS ET AL: "Expression profiles of hydrophobic surfactant proteins in children with diffuse chronic lung disease", RESPIRATORY RESEARCH, BIOMED CENTRAL LTD., LONDON, GB, vol. 6, no. 80, 22 July 2005 (2005-07-22), pages 1-11, XP021000173, ISSN: 1465-9921, DOI: 10.1186/1465-9921-6-80

## Description

### FIELD OF THE INVENTION

The present invention relates to methods and combinations for assessing the severity of chronic obstructive pulmonary disease.

### BACKGROUND OF THE INVENTION

Chronic obstructive pulmonary disease (COPD) is a major cause of morbidity and mortality across the world. It is the third leading cause of death in the United States, affecting over 16 million people in the US, and the fourth leading cause of death in developed countries. Continuing increases in COPD prevalence and mortality are anticipated in the next decades. COPD is a chronic inflammatory disorder of the lungs characterized by airflow limitation that is not fully reversible. The main risk factor for COPD is active smoking. COPD develops slowly and includes the development of emphysema, which is characterized by destruction and enlargement of alveoli and chronic bronchitis. It is further associated with cough and phlegm and small airway disease. In its stable state COPD is treated with bronchodilatators, anticholinergic agents and steroids inhalated or systemic. Theophylline and oxygen are also used and smoking cessation is highly recommended.

Exacerbations of COPD are a common feature and are clinically recognized by increased dyspnoe, cough and change of the amount of sputum. Accompanying signs of illness include fever and myalgias (Reilly, J.J., et al., p 1640, in: Harrison Principles of Internal Medicine, 17th Ed, p 1635 et seq). Currently exacerbations of COPD are treated according to an algorithm based on clinical symptoms (mild, moderate or severe) and the presence of risk factors (Sethi, S., et al NEJM (2008) 359, 2355-2365). Moreover there is ongoing discussion on which patients require hospitalisation and who can be kept as an outpatient (Niewoehner, D.E., NEJM (2010) 362, 1407 - 1416). Further, a major barrier to the assessment of patients is a lack of modifiable biomarkers that can be used as surrogates of clinical outcomes such as exacerbation and mortality.

In recent years it has been learned that exacerbations of COPD are caused by viral and bacterial infections and clinical manifestations are the result of infection itself and of the host response (Sethi et al., supra). Predominant infectious agents are *H. influenzae* and *Streptococcus pneumoniae* as bacteria, and rhinoviruses as viruses. COPD exacerbations affect regularly large and small airways but may also involve alveoli including type 2 pneumocytes which are the main source of surfactant proteins. Moreover infections causing exacerbations of COPD may become systemic and proceed to sepsis (Sethi, S., et al., supra). In fact, recent studies indicate that both natriuretic peptides (NT-pro BNP) and troponin T represent markers for poor outcome at 30 days but not at 1 year in patients with COPD exacerbations (Chang C.L. et al., Thorax (2011) 66, 764-768). The pathophysiological basis for the observed phenomenon has not been elucidated beyond the presence and importance of cardiac involvement in COPD.

In International application WO2006/097244 markers for COPD are disclosed, such as SpB. The document is silent about using samples from humans that do not have a history of heart failure.

Hence, there exists a need for means to further assess disease stage and to stratify patients with exacerbated COPD in order to stratify patients in appropriate subgroups in order to provide them with adequate care. Accordingly it is an object of the present invention to provide a method of assessing exacerbation of COPD and/or of stratifying patients for therapy of COPD.

### SUMMARY OF THE INVENTION

The methods and uses of the present invention are generally carried out *in vitro.* Each of the methods and uses defined below may consist of the step or steps described. Each of the methods and uses defined below may include the indicated steps and in addition include further steps. Samples from a patient, which may also be referred to as test samples, on which a method or use defined herein is carried out may have been obtained by a physician. The methods and uses defined in the following may be carried using any suitable equipment in any desired setting such as a laboratory. Typically results of the method will be reviewed or analysed by a physician for diagnostic purposes.

The invention in its broadest form is defined by independent claims 1 and 7:
1. A method of determining the severity of chronic obstructive pulmonary disease in a subject, the method comprising detecting the amount of pro-Pulmonary surfactant associated protein B (proSP-B), or an effective portion thereof, in a sample from the subject, wherein an increased amount of proSP-B, or the portion thereof, relative to a threshold value, indicates an elevated severity of chronic obstructive pulmonary disease, wherein the sample is a blood sample from a human who does not have a history of heart failure, and wherein a value above 35 pg/ml proSP-B and a value above 65 pg/ml of the portion of proSP-B defined by amino acid positions 285 to 334 of the sequence of SEQ ID NO: 1 indicates increased severity of chronic obstructive pulmonary disease.
7. The in-vitro use of a binding agent specific for proSP-B, or an effective portion thereof, for assessing the severity of chronic obstructive pulmonary disease in a subject, the use comprising the detection of the amount of proSP-B, or a portion thereof, in a blood sample from a human by means of the binding agent, wherein the human does not have a history of heart failure, wherein a value above about 35 pg/ml proSP-B and a value above 65 pg/ml of the portion of proSP-B defined by amino acid positions 285 to 334 of the sequence of SEQ ID NO: 1 indicates increased severity of chronic obstructive pulmonary disease.

Preferred embodiments are defined in dependent claims 2-6.

### BRIEF DESCRIPTION OF THE DRAWINGS

The invention will be better understood with reference to the detailed description when considered in conjunction with the non-limiting examples and the accompanying drawings.
**Figure 1** depicts an overview of levels of NT-proBNP, proSP-B, the C-terminal fragment of proSP-B, and CRP of patients with exacerbated COPD without (Group A, 32 individuals) and with (Group B, 32 individuals) systemic inflammatory response syndrome (SIRS) used in an exemplary study.
**Figure 2** shows the individual data of the 32 COPD patients with exacerbated COPD without SIRS of whom Fig. 1 presents an overview.
**Figure 3** shows the individual data of the 32 COPD patients with exacerbated COPD with SIRS of whom Fig. 1 presents an overview.
**Figure 4** depicts data of a group of control subjects (i) without cardiovascular disease (group 1), (ii) with cardiovascular disease and no indication of clinical heart failure (group 1) and (iii) cardiovascular disease and clinical heart failure, used in the exemplary study (group 3).
**Figure 5** shows the correlation of values of NT-pro BNP to values of proSP-B.
**Figure 6** shows the correlation of values of proSP-B to values of the C-terminal fragment of proSP-B.
**Figure 7** depicts the correlation of values obtained using the sensitive Troponin T test (Roche Diagnostics) and values of proSP-B.
**Figure 8** depicts the correlation of values obtained using the sensitive Troponin T test (Roche Diagnostics) and values ofNT-proBNP.

### DETAILED DESCRIPTION OF THE INVENTION

The present invention is based on the surprising finding that an exacerbation of COPD can be diagnosed on the basis of proSP-B levels in the subject. Without being bound by theory it is assumed that thereby the involvement of small bronchia and alveoli in severe obstructive pulmonary disease is assessed.

One method of the invention is a method of assessing the severity of COPD in a subject. An increase in severity of COPD is also referred herein as an exacerbation of COPD. An exacerbation of COPD is difficult to assess and this renders treatment choices and type of care extremely difficult. Both NT-proBNP and troponin T have been recognized as important biomarkers for the severity of COPD and outcome. However, it has remained unclear whether these biomarkers - herein also simply referred to as markers - represent underlying disease of sign of complications of COPD (Chang et al, 2011, supra). The present aspect of the invention adds an important piece to the evaluation of such patients: the involvement of small airways and alveoli (not recognized by standard imaging techniques). This aspect improves the assessment of exacerbated COPD and facilitates decision making with regard to type of care and type and extent of treatment.

As used herein, the term "increase" in severity of obstructive pulmonary disease is used on a relative basis, i.e. in comparison to the symptoms and severity of the disease known from a certain subject. As used herein the term "increase in severity of obstructive pulmonary disease" typically refers to an exacerbation of obstructive pulmonary disease, in particular an exacerbation of chronic obstructive pulmonary disease, such as an acute exacerbation of obstructive pulmonary disease, including an acute exacerbation of chronic obstructive pulmonary disease. Accordingly, determining the severity of obstructive pulmonary disease in some embodiments of any method or use according to the present invention refers to determining whether an exacerbation of chronic obstructive pulmonary disease has occurred. An exacerbation is known in the art as an increase in severity of a disease, in particular of a chronic disease. An exacerbation of obstructive pulmonary disease is accordingly a deterioration in obstructive pulmonary disease. Typically an exacerbation is an acute increase in severity of chronic obstructive pulmonary disease or an acute deterioration in obstructive pulmonary disease. Generally an exacerbation of obstructive pulmonary disease requires a change in therapy. As also explained in more detail in the following on an increase in severity of obstructive pulmonary disease, an exacerbation of obstructive pulmonary disease is typically accompanied by an increase in cough, sputum and dyspnoea. Exacerbation of obstructive pulmonary disease is treated by administering a bronchodilator and/or systemic application of a corticosteroid. In case of a strong increase in severity of symptoms administration of oxygen may be required.

The severity of a stable obstructive pulmonary disease, in contrast to an acute increase in severity, is furthermore in the art classified according to the GOLD score (cf. e.g. Harrison, Principles of Internal Medicine, 17th Ed. (Table 254-1), chapter 254). Criteria for assessing severity of obstructive pulmonary disease according to this classification in terms of lung function include the ratio of forced expiratory volume in one second (FEV₁) and forced vital capacity (FVC) as well as the predicted FEV₁ to determine the FEV₁ in % of predicted. In terms of clinical signs cough and sputum production are used to classify severity of the disease. As a general orientation, the following classification is applied: Stage I: Smoker's cough, little or no shortness of breath, no clinical signs of COPD, FEV₁ greater than 80% of predicted. Stage II: Shortness of breath on exertion, sputum-producing cough, some clinical signs of COPD, FEV₁ 50 - 80% of predicted. Stage III: Shortness of breath on mild exertion, FEV₁ 30 - 50% of predicted. Stage IV: Shortness of breath on mild exertion, right heart failure, cyanosis, FEV₁ less than 30% of predicted.

In the following a known subject is assumed to have mild obstructive pulmonary disease, which can be taken to be obstructive pulmonary disease of low severity. As also explained below, in such case obstructive pulmonary disease without increased severity manifests in the form of excessive cough and sputum production, usually accompanied by shortness of breath, i.e. dyspnoea. Obstructive pulmonary disease of low degree of severity is characterized by the presence of airflow obstruction, such as in the form of chronic bronchitis. Chronic bronchitis is defined by excessive secretion of bronchial mucus accompanied by daily cough over 3 or more months over a period of two or more consecutive years. Obstructive pulmonary disease progresses from a mild form via forms of higher severity up to a severe form, so that a mild form may also be referred to as an early stage, while a severe form may be referred to as a late stage. A severe form of obstructive pulmonary disease may involve a complication such as a systemic infection (see below).

Increased severity of obstructive pulmonary disease can be allocated to a worsening of the symptoms beyond day-to-day variations. In particular, increased severity of obstructive pulmonary disease is accompanied by increased dyspnoea, with dyspnoea being present at rest at degrees of highest severity. Further, typically an increased frequency or severity of cough occurs, and/or an increased sputum volume or a change in sputum character. Degrees of highest severity of obstructive pulmonary disease are further characterized by one or more of pneumonia, pulmonary hypertension, cor pulmonale as well as chronic respiratory failure.

Spirometry, which can be taken to be breath measurement, may be used to assess pulmonary function. Typically spirometry includes measuring the volume and/or flow of air that can be inhaled and exhaled. In obstructive pulmonary disease of low severity, an abnormal closing volume and reduced midexpiratory flow rate can be determined. At higher degrees of severity, a reduction in forced expiratory volume can be observed. Forced vital capacity is markedly reduced in high degrees of severity of obstructive pulmonary disease. Further, a reduction in the ratio of forced expiratory volume to vital capacity can be determined in stages of higher severity of the disease. In high degrees of severity of obstructive pulmonary disease generally air trapping occurs, which results in an increase of the ratio of residual volume to total lung capacity. Further, an increase in residual volume and an increase in total lung capacity occurs in stages of high severity of the disease.

Depending on whether chronic bronchitis dominates or not, the symptoms of the obstructive pulmonary disease in a subject with elevated severity of the disease, in the art two patterns of symptoms in COPD are distinguished, namely Type A, known as a "pink puffer", and type B, known as a "blue coater". In type A, chronic bronchitis is not the predominant conspicuity noticed by a subject. As indicated above, dyspnoea is at low severity of the disease the patient's major complaint in type A. Levels of haemoglobin are generally normal, i.e. 120-150 g/l, and Sa_{O2} at rest is at normal levels. Pa_{O2} is generally in the range from 65 to 75 mm Hg (about 8500 to 1000 Pa) and Pa_{CO2} is in the range from 35 to 40 mm Hg (about 4500 to about 5300). Hyperinflation with flattened diaphragms is detectable in chest radiography. Total lung capacity is generally increased, possibly markedly increased. Static lung compliance, i.e. the change in volume for a given applied pressure, is typically increased. Mild to moderate nocturnal oxygen desaturation can be observed, which is typically not associated with obstructive sleep apnoea. In type B chronic cough, typically accompanied by a mild dyspnoea is the predominant complaint of a subject. Often peripheral oedema occurs. Levels of haemoglobin are generally increased to values of about 150-180 g/l, and Pa_{CO2} is generally elevated, at values of about 50 to 60 mm Hg (about 6600 to about 8000 Pa). Increased interstitial markings are typically visible in chest radiography, while typically no flattening of diaphragms occurs. Total lung capacity is generally normal, possibly slightly increased. Static lung compliance is typically normal. Severe nocturnal oxygen desaturation can be observed, often associated with obstructive sleep apnoea.

A method of assessing the severity of obstructive pulmonary disease according to the present invention involves analysis of a sample from the subject *in vitro.* Typically the sample is or includes body fluid from the subject. The sample may in some embodiments be one of a whole blood sample, a plasma sample, a lymph sample and a serum sample. In some embodiments the method may include providing a sample from the subject. The sample may have been taken at any desired point in time before carrying out the method of the invention. Generally the time interval between taking the sample and carrying out the method of the invention is selected to allow analysis of intact proSP-B or an effective portion of proSPB. In some embodiments the sample has been taken on the same day or within the last two days such as the day before. The sample may for instance have been obtained 72 hours or less, such as 60 hours or less, 48 hours or less, 32 hours, 24 hours, 12 hours before the method of the invention is being carried out and been kept at about room temperature, i.e. about 18 °C. The sample may also have been kept at a temperature below room temperature, for instance be kept at a temperature in the range from about 2 °C to about 18 °C, such as from about 4 °C to about 18 °C, including at about 15 °C or below, or at or below 10 °C, such as about 4 °C or about 8 °C. In some embodiments the sample has been taken within the same week, such as 7 days or less, 6 days or less, 5 days or less, 4 days or less, 3 days or less, 2 days or less, 32 hours or less, 24 hours or less, 12 hours or less before the method of the invention is being carried out and been kept in fluid form at a temperature of about 12 °C or less, such as about 10 °C or less, about 8 °C or less, including e.g. about 4°C. The subject from which/whom the sample has been obtained is generally a mammal such as a rabbit, a mouse, a rat, a Guinea pig, a hamster, a dog, a pig, a cow, a goat, a sheep, a horse, a macaque, a gibbon or a human.

The word "about" as used herein refers to a value being within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. The term "about" is also used to indicate that the amount or value in question may be the value designated or some other value that is approximately the same. The phrase is intended to convey that similar values promote equivalent results or effects according to the invention. In this context "about" may refer to a range above and below of up to 10%, such as up to 5%, up to 2%, up to 1%, up to 0.5 % or up to 0.1 % of a given value.

In some embodiments the sample from the individual is a frozen sample. Generally the sample is frozen within the above detailed time intervals, e.g. 0 to about 48 or 0 to about 42 hours, and/or at the above exemplified time points, such as about 48 hours, about 36 hours or less, after the sample has been obtained from the individual and kept at room temperature. Where the sample is at least essentially void of cells such as in case of a serum or a plasma sample, the sample may be brought to a temperature below freezing point. Where the sample includes cells that are not intended to be depleted from the sample it may in some embodiments be desired to maintain these cells at least essentially intact for carrying out a method according to the invention. In such an embodiment a frozen sample may be formed by freezing an obtained sample after adding a cryoprotective agent such as DMSO, glycerol and/or hydroxyethyl starch. As an illustrative example DMSO may be used in a final concentration in the range from about 2% to about 10 %, such as about 2%, about 4%, about 5% or about 10% DMSO. Typically the sample is then frozen at a controlled rate to a temperature less than -50°C, whereafter the sample may for instance be stored, including long-term storage, at a temperature below -130°C such as -160°C, e.g. in liquid nitrogen for extended periods of time. Where the sample is a sample from which cells have at least essentially been removed, the sample may also be stored at a temperature below 0 °C without addition of a cryoprotective agent. The sample may for example be brought to a temperature of about -5 °C, about -10 °C, about -20 °C, about -50 °C, about -80 °C or below.

As indicated above, in some embodiments the sample may be depleted of cells. Depletion of cells from the sample may in some embodiments include general cell removal techniques such as centrifugation, filtration or cell chromatography. In some embodiments of a method according to the invention a sample as provided from the individual is depleted of erythrocytes, in some embodiments at least essentially cleared of erythrocytes, if required. Depletion or removal of erythrocytes may for example in some embodiments be required in case the sample is a whole blood sample or a blood cell sample. Lysis of erythrocytes may be carried out osmotically or chemically. Osmotic lysis is suitable in the contest of the present invention since erythrocytes lyse at an osmolarity at which leukocytes remain intact. In the art typically a solution of ammonium chloride is used for osmotic lysis, which may further include potassium bicarbonate and/or EDTA. A commercially available reagent may be used, such as the FCM Lysing solution by Santa Cruz (order no sc-3621), Erythrolyse Red Blood Cell Lysing Buffer by AbD Serotec or RBC Lysis Solution by 5 PRIME. Chemical lysis of erythrocytes may for example be achieved using an organic solvent such as diethylether or chloroform, and/or a surfactant, a copper containing solution or via adding one of certain bacterial or animal toxins. After lysis of erythrocytes the remaining blood cells may be collected, for example by means of centrifugation. Depletion or removal of cells may in some embodiments be carried out within a time interval of about 12 hours or less after the sample has been obtained from a subject, such as within about 8 hours, within about 6 hours, within about 5 hours, within about 4 hours, within about 3 hours, within about 2 hours, within about one hour or less.

A method according to the invention includes detecting the amount of pro-Pulmonary surfactant-associated protein B (proSP-B), or an effective portion thereof, which may include the C-terminal portion of proSPB (see below), in the sample. The protein proSP-B may be any respective variant or isoform of the respective species, e.g. human. The protein may for example be the human protein of the Swissprot/Uniprot accession number P07988 (version 147 as of 22 February 2012), corresponding to SEQ ID NO: 1. This protein may for instance be encoded by the mRNA of GenBank accession number NM_198843 (version NM_198843.2; GI:288856296) or the mRNA of GenBank accession number NM_000542 (version NM_000542.3; GI: 288856298). The corresponding gene may in some embodiments be the human gene SFTPB with the GenBank Gene ID: 6439 (as of 04 February 2012). The protein may in some embodiments be the rabbit protein of the Swissprot/ Uniprot accession number P15285 (version 96 as of 19 October 2011). The protein may also be the rat protein of the Swissprot/Uniprot accession number P22355 (version 93 as of 21 September 2011). The protein may in some embodiments be encoded by the rat gene SFTPB with the GenBank Gene ID: 192155 (as of 04 February 2012). In some embodiments the protein may be the bovine protein of the Swissprot/Uniprot accession number P15781 (version 88 as of 21 September 2011). The protein may in some embodiments be encoded by the bovine gene SFTPB with the GenBank Gene ID: 507398 (as of 28 February 2012). The protein may also be the mouse protein of the Swissprot/Uniprot accession number P50405 (version 89 as of 16 November 2011). In some embodiments the protein may be the dog protein of the Swissprot/Uniprot accession number P17129 (version 90 as of 21 September 2011). The protein may in some embodiments be the sheep protein of the Swissprot/Uniprot accession number Q9TU81 (version 40 as of 5 October 2011). The protein may also be the porcine protein of the Swissprot/Uniprot accession number P15782 (version 73 as of 27 July 2011). In some embodiments the protein may be the Guinea pig protein of the Swissprot/Uniprot accession number 035489 (version 53 as of 21 September 2011). Several natural variants of the human proSP-B protein of Swissprot/Uniprot accession number P07988 are known to exist, due to single nucleotide polymorphism. Such variants are listed under Swissprot/Uniprot entry P07988 as e.g. VAR_006948, VAR_013099, VAR_006950, VAR_006949, VAR_036856 and VAR_013100. The term proSP-B as used herein is meant to include such variants.

The term "variant" as used herein refers to a peptide or protein with an amino acid sequence which differs from the protein at issue due to one or more amino acid substitution(s), deletion(s) and/or addition(s). A variant may have an amino acid sequence with at least about 60 %, at least about 70 %, at least about 75 %, at least about 80 %, at least about 85 %, at least about 90 %, at least about 92 %, at least about 95 %, at least about 96 %, at least about 97 %, at least about 98 % or at least about 99 % sequence identity with a corresponding naturally occurring form of the respective protein. A variant may be an allelic variant or any other species specific homolog, paralog, or ortholog. A variant may also be a fragments of the corresponding protein, e.g. proSP-B, including a fragment of the aforementioned types of variants as long as these fragments have the essential immunological and biological properties as the known predominant form of the corresponding naturally occurring protein. A variant may for instance have immunological properties (i.e. epitope composition) comparable to those of a known protein form. Thus, a variant can typically be recognized by a binding partner used for determination of the concentration of the protein. A fragment is in some embodiments a degradation products of the known form of the protein, e.g. proSP-B. A variant may also differ due to posttranslational modifications such as phosphorylation or myristylation or have a different glycosylation pattern.

"Percent (%) sequence identity" with respect amino acid sequences disclosed herein is defined as the percentage of amino acid residues in a candidate sequence that are identical with the amino acid residues in a reference sequence, e.g. a proSP-B molecule, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publically available computer software such as BLAST, ALIGN, or Megalign (DNASTAR) software. Those skilled in the art can determine appropriate parameters for measuring alignment, including any algorithms needed to achieve maximum alignment over the full length of the sequences being compared. The same is true for nucleotide sequences disclosed herein.

Mature SP-B is secreted into the alveolar space where it is mainly found as a homodimer. SP-B is a protein that is included in lung surfactant, a complex mixture of proteins and lipids. SP-B is essential for lung surfactant function, including the regulation of surface tension in the lungs during breathing. Lung surfactant forms a continuous film at the liquid-air interface of the alveoli, which is fundamental to breathing. It allows normal breathing by reducing the surface tension at the air/water interface in alveoli. It further provides the first line of defense against inhaled microbes in the lungs. SP-B enhances the rate of phospholipid adsorption to the air/water interface, decreases surface tension by interfering with the attractive forces acting between water molecules and has anti-inflammatory properties. SP-B is thought to promote the rapid formation of the alveolar film. Sp-B is amphiphatic and to a large extent hydrophobic.

Hydrophobic ("water-fearing") matter, has a tendency to separate from water. In contrast thereto, hydrophilic ("water-loving") matter generally contains molecules which can form dipole-dipole interactions with water molecules and thus have a high wettability for water (see also below). Usually hydrophobic matter is apolar and possesses an even distribution of electron density. A related term is the indication lipophilic ("fat-loving"). Lipophilic matter attracts non-polar organic compounds, such as oils, fats, or greases. It is understood that on a general basis the terms "hydrophobic" and "lipophilic" are not necessarily synonymous. For example perfluorocarbon compounds are both hydrophobic and oleophobic, i.e. lack an affinity for oils. Such compounds accordingly have a tendency to separate from both water and hydrocarbons (though the latter to a lesser extent than from water). However, in the context of the present invention, "hydrophobic" and "lipophilic" can generally be used interchangeably. An amphiphilic, also called amphiphiphatic, compound has both hydrophilic and hydrophobic properties. Generally such a compound has hydrophilic portions and hydrophobic portions.

The mature form of surfactant-associated protein B differs from proSP-B in the lack of an N-terminal signal peptide, the lack of a subsequent N-terminal propeptide and the lack of a C-terminal propeptide. Further, proSP-B is being glycosylated, whereas the corresponding glycosylation sites are not present in mature SP-B. Mature human SP-B has a length of 70 amino acids, while human proSP-B has an additional 176 N-terminal amino acids and additional 102 C-terminal amino acids.

An effective portion of proSP-B is a peptide that has, including consists of, a sequence of amino acids that defines the C-terminal portion of proSPB, such as the C-terminal fourth of proSPB. It is recalled that the C-terminus of proSPB differs from the C-terminus of mature SPB in that proSPB has a propeptide sequence, which defines amino acids 280-381 of the human protein of the Swissprot/Uniprot accession number P07988. In typical embodiments an effective portion of proSPB has a portion of at least about 25, including at least about 30, at least about 35, at least about 40, at least about 45 or at least about 50 amino acids of the Saposin B-type 3 domain, which is defined by amino acids 295-370 of Swissprot/Uniprot accession number P07988. Typically a respective sequence of amino acids is located within the N-terminal half of the Saposin B-type 3 domain. Typically an effective portion of proSPB further includes 5 or more, including 10 or more amino acids of the C-terminal end of the Saposin B-type 2 domain of proSPB, which is defined by the amino acid positions corresponding to amino acids 204-281 of the sequence of Swissprot/Uniprot accession number P07988. In one embodiment an effective portion of proSPB includes, including consists of, the amino acid positions that correspond to amino acid positions 279 to 381 of the sequence of Swissprot/ Uniprot accession number P07988. In one embodiment an effective portion of proSPB includes, including consists of, the amino acid positions that correspond to amino acid positions 285 to 334 of the sequence of Swissprot/Uniprot accession number P07988. In some embodiments the effective portion of proSPB is monomeric. In some embodiments the effective portion of proSPB is dimeric, for example defining a homodimer.

One method of the invention is a method of diagnosing or aiding in the diagnosis of a condition associated with exacerbation of chronic obstructive pulmonary disease in a subject. The term exacerbation is meant to have its ordinary meaning, referring to worsening, deterioration, escalation, exacerbation or flare of the disease (supra), and can be used interchangeably with these words. The terms "diagnosing" and "diagnosis" as used herein refer to the assessment of the presence of absence of a medically relevant condition and in particular mean the process of identifying a medical condition on the basis of the results of a diagnostic procedure, including by by its signs and symptoms. Diagnosis may include processes such as screening for the predisposition for a medically relevant condition such as worsening of obstructive pulmonary disease, screening for the indication of a medically relevant condition, screening for a medically relevant condition and/or clinical or pathological diagnosis of a medically relevant condition. Generally the subject suffers from an increased symptomatology of the disease, in particular distress. An increase in severity of chronic obstructive pulmonary disease may be associated with a bacterial or viral infection. A related method of the invention is a method of diagnosing or aiding in the diagnosis of an elevated severity of obstructive pulmonary disease. It is understood that a respective diagnosis/ assessment involves a valuation which may subsequently turn out to be less than 100 % precise for a given individual. Such assessment is in some embodiments to be taken as an indication of the balance of probabilities rather than as a solid predication.

In some embodiments of any method according to the invention the subject from whom/which the sample originates, e.g. the sample is or has been obtained, is known not to suffer from a further lung disorder or lung damage (e.g. alveolo-capillary membrane damage) other than obstructive pulmonary disease. In some embodiments the subject is known not to suffer from pneumonia, from sepsis, massive transfusion, multiple trauma and/or pancreatitis. In some embodiments the subject is known not to suffer from the on-set of an autoimmune condition such as post-transplant lung rejection or an infection resulting in an inflammatory response. In some embodiments the subject is known not to have aspired gastric contents. In some embodiments the subject is known not to have been exposed to a noxious or toxic gas (for example a solvent or fume), cigarette smoke or dust, a herbicide or a pharmaceutical compound that is toxic to the lung such as Methothrexat or Bleomycin. In some embodiments the subject is known not to undergo radiation therapy of the lung or of an adjacent organ. In some embodiments the subject is known not to suffer from an infection that can lead to lung damage such as a systemic infection not associated with obstructive pulmonary disease, such as urosepsis. In some embodiments the subject is known not to suffer from an acute lung injury, including acute or adult respiratory distress syndrome ("ARDS"). In some embodiments the subject is known not to suffer from anoxia, including mountain sickness. In some embodiments the subject is known not to suffer from cardiac insufficiency or from an acute cardial incident such as acute coronary syndrome, angina pectoris, myocardial infarction.

In a method according to the invention the amount of proSP-B, or an effective portion thereof, is detected in the sample. The word "detect" or "detecting" when used herein in combination with the word "amount" is understood to generally refer to a quantitative rather than on a qualitative level. In this regard the words "value," "amount", "level" and "concentration" are used interchangeably herein. Accordingly, the method includes a quantification of proSP-B, i.e. the level of proSP-B is determined. Such a measurement may for instance rely on spectroscopic (e.g., NMR), surface plasmon resonance, photochemical, photometric, fluorometric, radiological, enzymatic or thermodynamic means. The measurement used is generally selected to be of a sensitivity that allows detection of proSP-B at levels in the range of the threshold value, in particular of a sensitivity that allows determining whether proSP-B levels are above or below the threshold value. Typically a binding partner of proSP-B may be contacted with the sample. In some embodiments a complex is allowed to form between proSP-B and the binding partner. The amount of the binding partner binding to, e.g. forming a complex with, proSP-B may be quantified in order to assess the amount of proSP-B present. Before detecting the amount of binding partner non-bound binding partner may in some embodiments be removed from the sample. A respective binding partner of proSP-B may be used in combination with a detectable marker.

Such a binding partner of proSP-B has a detectable affinity and specificity for proSP-B. Typically, binding is considered specific when the binding affinity is higher than 10⁻⁶ M. A binding partner of proSP-B has in some embodiments an affinity of about 10⁻⁸ M or higher or of about 10⁻⁹ M or higher. The term "specific" is understood to indicate that the binding partner is directed against, binds to, or reacts with proSP-B. Thus, being directed to, binding to or reacting with includes that the binding partner specifically binds to proSP-B. The term "specifically" in this context means that the binding partner reacts with proSP-B or/and a portion thereof, but at least essentially not with another protein. The term "another protein" includes any protein, including proteins closely related to or being homologous to proSP-B against which the binding partner is directed to. The term "does not essentially bind" means that the binding partner does not bind another protein, i.e., shows a cross-reactivity of less than about 30%, such as less than about 20%, less than about 10%, including less than about 9, 8, 7, 6 or 5%, when compared to the affinity to proSP-B. Whether the binding partner specifically reacts as defined herein above can easily be tested, inter alia, by comparing the reaction of a respective binding partner with proSP-B and the reaction of the binding partner with (an) other protein(s). "Specific binding" can also be determined, for example, in accordance with Western blot analysis, ELISA-, RIA-, ECL-, IRMA-tests, FACS, IHC and peptide scans.

A respective binding partner of proSP-B may be an immunoglobulin, a fragment thereof, a proteinaceous binding molecule with immunoglobulin-like functions or an aptamer. Examples of (recombinant) immunoglobulin fragments are Fab fragments, Fv fragments, single-chain Fv fragments (scFv), diabodies or domain antibodies (Holt, L.J., et al., Trends Biotechnol. (2003), 21, 11, 484-490). An example of a proteinaceous binding molecule with immunoglobulin-like functions is a mutein based on a polypeptide of the lipocalin family (WO 03/029462, Beste et al., Proc. Natl. Acad. Sci. USA (1999) 96, 1898-1903). Lipocalins, such as the bilin binding protein, the human neutrophil gelatinase-associated lipocalin, human Apolipoprotein D or glycodelin, possess natural ligand-binding sites that can be modified so that they bind to selected small protein regions known as haptens. Examples of other proteinaceous binding molecules are the so-called glubodies (see WO 96/23879), proteins based on the ankyrin scaffold (Mosavi, L.K., et al., Protein Science (2004) 13, 6, 1435-1448) or crystalline scaffold (WO 01/04144) the proteins described in Skerra, J Mol. Recognit. (2000) 13, 167-187, a protein based on a plurality of low-density lipoprotein receptor class A (LDLR-A) domains, an AdNectin, a tetranectin and an avimer. Avimers contain so called A-domains that occur as strings of multiple domains in several cell surface receptors (Silverman, J, et al., Nature Biotechnology (2005) 23, 1556-1561). A molecule that forms a complex with a binding partner of proSP-B may likewise be an immunoglobulin, a fragment thereof or a proteinaceous binding molecule with immunoglobulin-like functions, as explained above. Thus, in an exemplary embodiment detecting the amount of proSP-B may be carried out using a first antibody or antibody fragment capable of specifically binding proSP-B, as well as a second antibody or antibody fragment capable of specifically binding the first antibody or antibody fragment.

The term "antibody" as used herein, is understood to include an immunoglobulin and an immunoglobulin fragment that is capable of specifically binding a selected protein, e.g. proSP-B, as well as a respective proteinaceous binding molecule with immunoglobulin-like functions. As an illustrative example an antibody may be a camel heavy chain immunoglobulin. As a few further non-limiting examples, an antibody may be an EGF-like domain, a Kringle-domain, a fibronectin type I domain, a fibronectin type II domain, a fibronectin type III domain, a PAN domain, a G1a domain, a SRCR domain, a Kunitz/Bovine pancreatic trypsin Inhibitor domain, tendamistat, a Kazal-type serine protease inhibitor domain, a Trefoil (P-type) domain, a von Willebrand factor type C domain, an Anaphylatoxin-like domain, a CUB domain, a thyroglobulin type I repeat, an LDL-receptor class A domain, a Sushi domain, a Link domain, a Thrombospondin type I domain, an immunoglobulin domain or a an immunoglobulin-like domain (see above for further examples). In some embodiments an antibody is an aptamer, including a Spiegelmer®, described in e.g. WO 01/92655. An aptamer is typically a nucleic acid molecule that can be selected from a random nucleic acid pool based on its ability to bind a selected other molecule such as a peptide, a protein, a nucleic acid molecule a or a cell. Aptamers, including Spiegelmers, are able to bind molecules such as peptides, proteins and low molecular weight compounds. Spiegelmers® are composed of L-isomers of natural oligonucleotides. Aptamers are engineered through repeated rounds of *in vitro* selection or through the SELEX (systematic evolution of ligands by exponential enrichment) technology. The affinity of Spiegelmers to their target molecules often lies in the pico- to nanomolar range and is thus comparable to immunoglobulins. An aptamer may also be a peptide. A peptide aptamer consists of a short variable peptide domain, attached at both ends to a protein scaffold.

By "fragment" in reference to a polypeptide such as an immunoglobulin or a proteinaceous binding molecule is meant any amino acid sequence present in a corresponding polypeptide, as long as it is shorter than the full length sequence and as long as it is capable of performing the function of interest of the protein - in the case of an immunoglobulin specifically binding to the desired target, e.g. antigen (proSP-B, for example). The term "immunoglobulin fragment" refers to a portion of an immunoglobulin, often the hypervariable region and portions of the surrounding heavy and light chains that displays specific binding affinity for a particular molecule. A hypervariable region is a portion of an immunoglobulin that physically binds to the polypeptide target.

An immunoglobulin may be monoclonal or polyclonal. The term "polyclonal" refers to immunoglobulins that are heterogenous populations of immunoglobulin molecules derived from the sera of animals immunized with an antigen or an antigenic functional derivative thereof. For the production of polyclonal immunoglobulins, one or more of various host animals may be immunized by injection with the antigen. Various adjuvants may be used to increase the immunological response, depending on the host species. "Monoclonal immunoglobulins" or "Monoclonal antibodies" are substantially homogenous populations of immunoglobulins to a particular antigen. They may be obtained by any technique which provides for the production of immunoglobulin molecules by continuous cell lines in culture. Monoclonal immunoglobulins may be obtained by methods well known to those skilled in the art (see for example, Köhler et al., Nature (1975) 256, 495-497, and U.S. Patent No. 4,376,110). An immunoglobulin or immunoglobulin fragment with specific binding affinity only for proSP-B can be isolated, enriched, or purified from a prokaryotic or eukaryotic organism. Routine methods known to those skilled in the art enable production of both immunoglobulins or immunoglobulin fragments and proteinaceous binding molecules with immunoglobulin-like functions, in both prokaryotic and eukaryotic organisms.

In more detail, an immunoglobulin may be isolated by comparing its binding affinity to a protein of interest, e.g. proSP-B, with its binding affinity to other polypeptides. Humanized forms of the antibodies of the present invention may be generated using one of the procedures known in the art such as chimerization or CDR grafting. In general, techniques for preparing monoclonal antibodies and hybridomas are well known in the art. Any animal such as a goat, a mouse or a rabbit that is known to produce antibodies can be immunized with the selected polypeptide, e.g. proSP-B. Methods for immunization are well known in the art. Such methods include subcutaneous or intraperitoneal injection of the polypeptide. One skilled in the art will recognize that the amount of polypeptide used for immunization and the immunization regimen will vary based on the animal which is immunized, including the species of mammal immunized, its immune status and the body weight of the mammal, as well as the antigenicity of the polypeptide and the site of injection.

The polypeptide may be modified or administered in an adjuvant in order to increase the peptide antigenicity. Methods of increasing the antigenicity of a polypeptide are well known in the art. Such procedures include coupling the antigen with a heterologous protein (such as globulin or β-galactosidase) or through the inclusion of an adjuvant during immunization.

Typically, the immunized mammals are bled and the serum from each blood sample is assayed for particular antibodies using appropriate screening assays. As an illustrative example, anti-proSP-B immunoglobulins may be identified by immunoprecipitation of ¹²⁵I-labeled cell lysates from proSP-B expressing cells (see, Sanchez-Madrid et al., 1986 and Hemler et al., 1987). Anti-proSP-B immunoglobulins may also be identified by flow cytometry, e.g., by measuring fluorescent staining of Ramos cells incubated with an antibody believed to recognize proSP-B.

For monoclonal immunoglobulins, lymphocytes, typically splenocytes, from the immunized animals are removed, fused with an immortal cell line, typically myeloma cells, such as SP2/0-Agl4 myeloma cells, and allowed to become monoclonal immunoglobulin producing hybridoma cells. Typically, the immortal cell line such as a myeloma cell line is derived from the same mammalian species as the lymphocytes. Illustrative immortal cell lines are mouse myeloma cell lines that are sensitive to culture medium containing hypoxanthine, aminopterin and thymidine ("HAT medium"). Typically, HAT-sensitive mouse myeloma cells are fused to mouse splenocytes using 1500 molecular weight polyethylene glycol ("PEG 1500"). Hybridoma cells resulting from the fusion may then be selected using HAT medium, which kills unfused and unproductively fused myeloma cells (unfused splenocytes die after several days because they are not transformed).

Any one of a number of methods well known in the art can be used to identify a hybridoma cell which produces an immunoglobulin with the desired characteristics. Typically the culture supernatants of the hybridoma cells are screened for immunoglobulins against the antigen. Suitable methods include, but are not limited to, screening the hybridomas with an ELISA assay, Western blot analysis, or radioimmunoassay (Lutz et al., Exp. Cell Res. [1988] 175, 109-124). Hybridomas prepared to produce anti-proSP-B immunoglobulins may for instance be screened by testing the hybridoma culture supernatant for secreted antibodies having the ability to bind to a recombinant proSP-B expressing cell line. To produce antibody homologs which are within the scope of the invention, including for example, anti-proSP-B antibody homologs, that are intact immunoglobulins, hybridoma cells that tested positive in such screening assays can be cultured in a nutrient medium under conditions and for a time sufficient to allow the hybridoma cells to secrete the monoclonal immunoglobulins into the culture medium. Tissue culture techniques and culture media suitable for hybridoma cells are well known in the art. The conditioned hybridoma culture supernatant may be collected and for instance the anti-proSP-B immunoglobulins optionally be further purified by well-known methods. Alternatively, the desired immunoglobulins may be produced by injecting the hybridoma cells into the peritoneal cavity of an unimmunized mouse. The hybridoma cells proliferate in the peritoneal cavity, secreting the immunoglobulin which accumulates as ascites fluid. The immunoglobulin may be harvested by withdrawing the ascites fluid from the peritoneal cavity with a syringe.

Hybridomas secreting the desired immunoglobulins are cloned and the class and subclass are determined using procedures known in the art. For polyclonal immunoglobulins, immunoglobulin containing antisera is isolated from the immunized animal and is screened for the presence of immunoglobulins with the desired specificity using one of the above-described procedures. The above-described antibodies may also be immobilized on a solid support. Examples of such solid supports include plastics such as polycarbonate, complex carbohydrates such as agarose and sepharose, acrylic resins and such as polyacrylamide and latex beads. Techniques for coupling antibodies to such solid supports are well known in the art.

A plurality of conventional display technologies is available to select an immunoglobulin, immunoglobulin fragment or proteinaceous binding molecule. Li et al. (Organic & Biomolecular Chemistry (2006), 4, 3420-3426) have for example demonstrated how a single-chain Fv fragment capable of forming a complex with a selected DNA adapter can be obtained using phage display. Display techniques for instance allow the generation of engineered immunoglobulins and ligands with high affinities for a selected target molecule. It is thus also possible to display an array of peptides or proteins that differ only slightly, typically by way of genetic engineering. Thereby it is possible to screen and subsequently evolve proteins or peptides in terms of properties of interaction and biophysical parameters. Iterative rounds of mutation and selection can be applied on an *in vitro* basis.

*In vitro* display technology for the selection of peptides and proteins relies on a physical linkage between the peptide or protein and a nucleic acid encoding the same. A large panel of techniques has been established for this purpose, with the most commonly used being phage/virus display, ribosome display, cell-surface display, 'peptides on plasmids', mRNA display, DNA display, and *in vitro* compartmentalisation including micro-bead display (for reviews see e.g. Rothe, A., et al., FASEB J. (2006) 20, 1599 -1610; Sergeeva, A., et al., Advanced Drug Delivery Reviews (2006) 58, 1622-1654).

Different means of physically linking a protein or peptide and a nucleic acid are also available. Expression in a cell with a cell surface molecule, expression as a fusion polypeptide with a viral/phage coat protein, a stabilised *in vitro* complex of an RNA molecule, the ribosome and the respective polypeptide, covalent coupling *in vitro* via a puromycin molecule or via micro-beads are examples of ways of linking the protein/peptide and the nucleic acid presently used in the art. A further display technique relies on a water-in-oil emulsion. The water droplets serve as compartments in each of which a single gene is transcribed and translated (Tawfik, D.S., & Griffiths, A.D., Nature Biotech. (1998) 16, 652-656, US patent application 2007/0105117). This physical linkage between the peptide or protein and the nucleic acid (encoding it) provides the possibility of recovering the nucleic acid encoding the selected protein or peptide. Compared to techniques such as immunoprecipitation, in display techniques thus not only binding partners of a selected target molecule can be identified or selected, but the nucleic acid of this binding partner can be recovered and used for further processing. Present display techniques thus provide means for e.g. target discovery, lead discovery and lead optimisation. Vast libraries of peptides or proteins, e.g. antibodies, potentially can be screened on a large scale.

As indicated above, a detectable marker may be coupled to a binding partner of proSP-B or a molecule that forms a complex with the binding partner of proSP-B. A respective detectable marker, which may be coupled to a binding partner of proSP-B or a molecule that forms a complex therewith, may be an optically detectable label, a fluorophore, or a chromophore. Examples of suitable labels include, but are not limited to, an organic molecule, an enzyme, a radioactive, fluorescent, and/or chromogenic moiety, a luminescent moiety, a hapten, digoxigenin, biotin, a metal complex, a metal and colloidal gold. Accordingly an excitable fluorescent dye, a radioactive label, included for instance in a radioactive amino acid, a fluorescent protein or an enzyme may for instance be used to detect e.g. the level of CD62L or the level of PSGL-1. Examples of suitable fluorescent dyes include, but are not limited to, fluorescein isothiocyanate, 5,6-carboxymethyl fluorescein, Cascade Blue®, Oregon Green®, Texas red, nitrobenz-2-oxa-1,3-diazol-4-yl, coumarin, dansyl chloride, rhodamine, aminomethyl coumarin, DAPI, Eosin, Erythrosin, BODIPY®, pyrene, lissamine, xanthene, acridine, an oxazine, phycoerythrin, a Cy dye such as Cy3, Cy3.5, Cy5, Cy5PE, Cy5.5, Cy7, Cy7PE or Cy7APC, an Alexa dye such as Alexa 647, and NBD (Naphthol basic dye). Examples of suitable fluorescent protein include, but are not limited to, EGFP, emerald, EYFP, a phycobiliprotein such as phycoerythrin (PE) or allophycocyanin, Monomeric Red Fluorescent Protein (mRFP), mOrange, mPlum and mCherry. In some embodiments a reversibly photoswitchable fluorescent protein such as Dronpa, bsDronpa and Padron may be employed (Andresen, M., et al., Nature Biotechnology (2008) 26, 9, 1035). Regarding suitable enzymes, alkaline phosphatase, soybean peroxidase, or horseradish peroxidase may serve as a few illustrative examples. Typical radioactive labels include, but are not limited to, ³⁵S, ¹²⁵I, ³²P, and ³³P. A radioactive label can be detected by any method known and appropriate such as a light-sensitive film or a phosphor imager. In some embodiments a method of detection may include electrophoresis, HPLC, flow cytometry, fluorescence correlation spectroscopy or a modified form of these techniques. Some or all of these steps may be part of an automated separation/detection system.

In some embodiments a method of detection may include electrophoresis, HPLC, flow cytometry, fluorescence correlation spectroscopy or a modified form of these techniques. Some or all of these steps may be part of an automated separation/detection system.

Determining the amount of proSP-B in the sample can be carried out by way of any suitable technique available. An illustrative example of a suitable technique in this regard is a radiolabel assay such as a Radioimmunoassay (RIA) or an enzyme-immunoassay such as an Enzyme Linked Immunoabsorbent Assay (ELISA), precipitation (particularly immunoprecipitation), a sandwich enzyme immune test, an electro-chemiluminescence sandwich immunoassay (ECLIA), a dissociation-enhanced lanthanide fluoro immuno assay (DELFIA), a scintillation proximity assay (SPA), turbidimetry, nephelometry, latex-enhanced turbidimetry or nephelometry, or a solid phase immune test. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described herein. While a RIA is based on the measurement of radioactivity associated with a complex formed between an immunoglobulin or a proteinaceous binding molecule with immunoglobulin-like functions and an antigen, an ELISA is based on the measurement of an enzymatic reaction associated with a complex formed between an immunoglobulin or a proteinaceous binding molecule with immune-globulin-like functions and an antigen. Typically a radiolabel assay or an enzyme-immunoassay involves one or more separation steps in which a binding partner of e.g. proSP-B that has not formed a complex with proSP-B is being removed, thereby leaving only binding partner of proSP-B behind, which has formed a complex with proSP-B. This allows the generation of specific signals originating from the presence of proSP-B.

An ELISA or RIA test can be competitive for measuring the amount of proSP-B, i.e. the amount of antigen. For example, an enzyme labeled antigen is mixed with a test sample containing antigen, which competes for a limited amount of immunoglobulin or a proteinaceous binding molecule with immunoglobulin-like functions. The reacted (bound) antigen is then separated from the free material, and its enzyme activity is estimated by addition of substrate. An alternative method for antigen measurement is the double immunoglobulin/proteinaceous binding molecule sandwich technique. In this modification a solid phase is coated with specific immunoglobulin or a proteinaceous binding molecule with immunoglobulin-like functions. This is then reacted with the sample from the subject that contains the antigen. Then enzyme labeled specific immunoglobulin/proteinaceous binding molecule is added, followed by the enzyme substrate. The 'antigen' in the test sample is thereby 'captured' and immobilized on to the sensitized solid phase where it can itself then immobilize the enzyme labeled immunoglobulin/proteinaceous binding molecule. This technique is analogous to the immunoradiometric assays.

In an indirect ELISA method, an antigen is immobilized by passive adsorption on to the solid phase. A test serum may then be incubated with the solid phase and any immunoglobulin in the test serum forms a complex with the antigen on the solid phase. Similarly a solution of a proteinaceous binding molecule with immunoglobulin-like functions may be incubated with the solid phase to allow the formation of a complex between the antigen on the solid phase and the proteinaceous binding molecule. After washing to remove unreacted serum components an anti-immunoglobulin immunoglobulin anti-proteinaceous binding molecule immunoglobulin, linked to an enzyme is contacted with the solid phase and incubated. Where the second reagent is selected to be a proteinaceous binding molecule with immunoglobulin-like functions, a respective proteinaceous binding molecule that specifically binds to the proteinaceous binding molecule or the immunoglobulin directed against the antigen is used. A complex of the second proteinaceous binding molecule or immunoglobulin and the first proteinaceous binding molecule or immunoglobulin, bound to the antigen, is formed. Washing again removes unreacted material. In the case of RIA radioactivity signals are being detected. In the case of ELISA the enzyme substrate is added. Its colour change will be a measure of the amount of the immobilized complex involving the antigen, which is proportional to the antibody level in the test sample.

In another embodiment the immunoglobulin or the proteinaceous binding molecule with immunoglobulin-like functions may be immobilized onto a surface, such as the surface of a polymer bead (supra), or coated onto the surface of a device such as a polymer plate or a glass plate. As a result the immune complexes can easily be separated from other components present by simply washing the surface, e.g. the beads or plate. This is the most common method currently used in the art and is referred to as solid phase RIA or ELISA. This embodiment may be particularly useful for determining the amount of proSP-B. On a general basis, in any embodiment of a radiolabel assay or of an enzyme-immunoassay passive adsorption to the solid phase can be used in the first step. Adsorption of other reagents can be prevented by inclusion of wetting agents in all the subsequent washing and incubation steps. It may be advantageous to perform washing to prevent carry-over of reagents from one step to the next.

Various other modifications of ELISA have been used in the art. For example, a system where the second proteinaceous binding molecule or immunoglobulin used in the double antibody sandwich method is from a different species, and this is then reacted with an anti-immunoglobulin enzyme conjugate or an anti-proteinaceous binding molecule enzyme conjugate. This technique comes with the potential advantage that it avoids the labeling of the specific immunoglobulin or proteinaceous binding molecule, which may be in short supply and of low potency. This same technique can be used to assay immunoglobulin or proteinaceous binding molecule where only an impure antigen is available; the specific reactive antigens are selected by the antibody immobilized on the solid phase.

In another example of an ELISA assay for antigen, a surface, a specific antigen is immobilized on a surface, e.g. a plate used, and the surface is then incubated with a mixture of reference immunoglobulins or proteinaceous binding molecules and a test sample. If there is no antigen in the test sample the reference immunoglobulin or proteinaceous binding molecule becomes fixed to an antigen sensitized surface. If there is antigen in the test solution this combines with the reference immunoglobulin or proteinaceous binding molecule, which cannot then react with the sensitized solid phase. The amount of immunoglobulin/proteinace-ous binding molecule attached is then indicated by an enzyme labeled anti-globulin/anti-binding molecule conjugate and enzyme substrate. The amount of inhibition of substrate degradation in the test sample (as compared with the reference system) is proportional to the amount of antigen in the test system.

As indicated above, determining the amount of proSP-B in the sample typically involves the formation of signals, e.g. signals generated by a detectable marker (supra) that can be quantified. Quantifying the signals in order to determine the amount of proSP-B in the sample may be carried out by comparing of obtained signals with those of one or more reference measurements. A respective reference measurement is generally based on the signal generated by a known amount of proSP-B. Such a known amount of proSP-B may for example be present in a sample with a composition that resembles the sample from the patient, in which the amount of proSP-B is to be determined. A respective reference sample may be taken to define an external reference sample. In some embodiments of a method of the invention an internal reference sample may in addition or alternatively be used. Such an internal reference sample is a sample obtained from the subject at a previous point of time. The amount of proSP-B in such a sample may be determined to identify the changes in proSP-B levels in the subject.

In the method according to the invention, e.g. a method of assessing the severity of obstructive pulmonary disease in a subject, the amount of proSP-B determined in the sample may be compared to a threshold value. Such a threshold value may in some embodiments be a predetermined threshold value. In some embodiments the threshold value is based on the amount of proSP-B in a control sample. A respective control sample may have any condition that varies from the sample main measurement itself. Such a control sample may be a sample of, or including, the corresponding body fluid as the sample from the subject. A control sample may for example be a sample, such as a blood sample, a plasma sample or a serum sample, of a subject known not to suffer from obstructive pulmonary disease. A control sample may also be from a subject known not to suffer from exacerbated obstructive pulmonary disease. In some embodiments a respective control sample is from a subject that is age-matched. In some embodiments a respective control sample is from a subject that is known not to have a confounding disease, in some embodiments from a subject known not to have a disease.

In some embodiments a threshold value is a collection of data of a plurality of control samples, which may also be referred to as a reference samples. In such embodiments the threshold value may be set to be a significant difference between the control and the sample from the subject of interest. The term "significant" is used to indicate that the level of increase is of statistical relevance. As an illustrative example a plurality of measurements, including a plurality of samples may have been obtained from the subject of interest. The p value may then be determined. A p value of 0.05, 0.02, 0.01 or lower may be taken to indicate a difference. In some embodiments a significant increase is a deviation of a value of a test sample relative to a value of a control sample of about 2 fold or more, including 3 fold or more, such as at least about 5 to about 10 fold or even more.

In some embodiments a threshold value is based on a control or reference value obtained concomitantly with the value of the sample from the subject. In some embodiments a respective control or reference value is determined at a different point in time, for example at a point in time earlier than the measurement of the sample from the subject is carried out. It is understood that the terms control and reference may in some embodiments be a range of values.

The comparison to a threshold value, which may be a predetermined threshold value, can be carried out manually, semi-automatically or in a fully automated manner. In some embodiments the comparison may be computer assisted. A computer assisted comparison may employ values stored in a database as a reference for comparing an obtained value or a determined amount, for example via a computer implemented algorithm. Likewise, a comparison to a reference measurement may be carried out manually, semi-automatically or in a fully automated manner, including in a computer assisted manner.

A predetermined threshold value may in some embodiments be set on the basis of data collected from patients known to suffer from COPD without exacerbation. In some embodiments a certain percentile of such data may be used as a threshold value. The range of the values of a set of data obtained from such patients can be divided into 100 equal parts, i.e. percentages of the range can be determined. A percentile represents the value within the respective range below which a certain percent of the data fall, in other words the percentage of the values that are smaller than that value. For example the 95th percentile is the value below which 95 percent of the data are found. In some embodiments a level of proSP-B may be regarded as increased or elevated if it is above the 90^{th} percentile, above the 92^{nd} percentile, above the 93^{rd} percentile, above the 94^{th} percentile, above the 95^{th} percentile, above the 96^{th} percentile, above the 97^{th} percentile, above the 98^{th} percentile or above the 99^{th} percentile.

In some embodiments the average value of data from patients known to suffer from COPD without exacerbation may be determined and used to determine a threshold value. As an illustrative example, the value of standard deviation or relative standard deviation, in some embodiments multiplied with a factor, which may be larger or smaller than 1. In some embodiments a multiple of standard deviation or relative standard deviation, such as 1.2, 1.5 or 2 times the standard deviation, may be added to the average value determined. In some embodiments establishing a predetermined threshold value includes collecting from patients known to suffer from COPD without exacerbation over a period of time, such as a plurality of months. Average proSP-B levels, standard deviation, and relative standard deviation at given times may be calculated for the patients to determine a range of proSP-B levels associated with COPD without exacerbation thereof. In some embodiments an average of a selected number, such as 2, 3, 4, 5 or more of the highest proSP-B levels determined over time may be used as a predetermined threshold value. In some embodiments the average value of all data collected at different time points may be determined and used to determine a threshold value, for instance by adding standard deviation, relative standard deviation or e.g. a multiple thereof (supra). When test result from a patient to be evaluated is collected, it will be compared to the threshold value. Statistical differences between the test result and the threshold value will be determined to identify significant variances in between.

The amount of proSP-B determined in or from a sample of a subject can be compared to a single control sample or a plurality of control samples, such as a sample from a control subject, in any suitable manner. As an illustrative example, the level of proSP-B in a control sample can be characterized by an average (mean) value coupled with a standard deviation value, for example at a given time point. In some embodiments the level of proSP-B in a subject may be considered increased when it is one standard deviation or more higher than the average value of the corresponding expression level determined in one or more control samples. In some embodiments the determined level of proSP-B is regarded as increased where the obtained value is about 1.5 standard deviations higher or lower, including about two, about three, about four or more standard deviations higher or lower than the average value determined in a control sample. In some embodiments the determined amount of proSP-B is regarded as different where the obtained value is about 1.2 times or more higher or lower, including about 1.5 times, about two fold, about 2.5-fold, about three fold, about 3.5 fold, about 4-fold, about 5-fold or more higher or lower than the protein level determined in a control sample. In some embodiments the determined level of proSP-B is regarded as increased where the obtained value is about 0.8-fold or less, including about 70 %, about 60 %, about 50 %, about 40 %, about 30 %, about 25 %, about 20 % or lower than the of proSP-B level determined in a control sample.

If for example a level of proSP-B is detected that is above a, e.g. predetermined, threshold value, this may indicate an increase in severity of obstructive pulmonary disease. A level of proSP-B above a threshold value may indicate a condition where the subject is suffering from severe exacerbated COPD. If a level of proSP-B is detected that is below a predetermined threshold value, this may indicate that no increase in severity of obstructive pulmonary disease has occurred.

The method described above can likewise be used to diagnose the severity of COPD in a subject (supra) that is suspected or known to suffer from an increase in severity of COPD. Detecting the amount of proSP-B and comparing the same to a threshold value may be carried out as above. A level of proSP-B above a predetermined threshold value may indicate a condition where the subject is suffering from severe exacerbated COPD. A level of proSP-B below a predetermined threshold value may in some embodiments indicate a condition where the subject is suffering from mild exacerbated COPD.

In a method according to the invention as described above the subject from whom/which the sample originates is generally known to have obstructive pulmonary disease. An increased amount of proSP-B, or the portion thereof, relative to the threshold value, indicates an increase in severity of obstructive pulmonary disease and vice versa. A method as described above may further in some embodiments be a method of assessing the chances of survival from obstructive pulmonary disease in a subject. The subject is generally known to have obstructive pulmonary disease. An increased amount of proSP-B, or the portion thereof, relative to the threshold value, may indicate low chances of survival of obstructive pulmonary disease.

In some embodiments the amount of proSP-B in a sample is being detected repeatedly. In some embodiments repeated detection is carried out on different samples from the same individual. In some embodiments a plurality of measurements is carried out on a plurality of samples from the same patient. In each of the samples the level of expression of proSP-B is determined. Typically the level of expression determined in each of the samples is compared to a threshold value as detailed above. In some embodiments the plurality of samples from the same individual is taken over a period of time at certain time intervals, including at predetermined time intervals. As an illustrative example, samples may be taken from the same subject at time intervals of about 72 hours, about 48 hours, about 32 hours, about 24 hours, about 18 hours, about 12 hours, about 8 hours, about 4 hours, about 2 hours, about 1 hour, about 45 minutes, about 30 minutes, about 25 minutes, about 20 minutes, about 15 minutes or less. Such an embodiment may be taken as a method of monitoring the amount of proSP-B. Matching samples may in some embodiments be used to determine a threshold value for each corresponding time point. The average value may be determined and the standard deviation calculated for each given time point. A value determined in the sample from the subject falling outside of the mean plus 1 standard deviation may for instance be indicative of an elevated severity of obstructive pulmonary disease.

Monitoring the amount of proSP-B may be included in the context of monitoring a therapy, for example in order to assess the efficacy thereof or to evaluate a subject's response to a certain treatment.

In embodiments where the sample is a human blood, plasma or serum sample from a patient without a history of heart failure, a suitable threshold value may be a value of about 35 ng/ml proSP-B. Likewise, for a human blood, plasma or serum sample from a patient without a history of heart failure a suitable threshold value may be a value of about 65 ng/ml of the portion of proSP-B that is defined by or that is corresponding to the amino acid positions 285 to 334 of the sequence of Swissprot/Uniprot accession number P07988. A value above about 35 ng/ml proSP-B, including above about 40 ng/ml, above about 45 ng/ml or above about 50 ng/ml proSP-B may indicate an increase in severity of obstructive pulmonary disease. A value above about 65 ng/ml, including above about 70 ng/ml, above about 75 ng/ml, above about 80 ng/ml, above about 85 ng/ml, above about 90 ng/ml, above about 95 ng/ml above about 100 ng/ml, above about 105 ng/ml, above about 110 ng/ml or above about 115 ng/ml of the respective effective portion of proSP-B may indicate an increase in severity of obstructive pulmonary disease. In this regard, a patient without a history of heart failure is either a patient who/which did not previously suffer from heart failure or a subject for whom/which no sufficient symptoms were known, which would allow diagnosing the occurrence of heart failure or which would indicate an assessment as to whether one or more processes/tests should be performed that allow a diagnosis of occurrence or non-occurrence of heart failure.

As explained above, obstructive pulmonary disease progresses from mild to severe forms. In this regard a general correlation between the severity of obstructive pulmonary disease and the level of proSP-B has been observed by the present inventors. Thus a higher level of proSP-B is in typical embodiments an indication of a higher severity of the disease. As an example, for a human blood, plasma or serum sample from a patient without a history of heart failure a high severity of the disease can be inferred from a value of proSP-B that is at or above about 100 ng/ml, such as at or above about 120 ng/ml, at or above about 140 ng/ml, at or above about 150 pg/ml, at or above about 160 pg/ml, at or above about 170 pg/ml, at or above about 180 pg/ml, at or above about 190 ng/ml or at or above about 200 ng/ml. Correspondingly, for a human blood, plasma or serum sample from a patient without a history of heart failure a high severity of the disease can be inferred from a value of the portion of proSP-B that is defined by or that is corresponding to the amino acid positions 285 to 334 of the sequence of Swissprot/Uniprot accession number P07988 that is at or above about 220 ng/ml, such as at or above about 250 ng/ml, at or above about 270 ng/ml, at or above about 300 ng/ml, at or above about 320 ng/ml, at or above about 340 ng/ml, at or above about 360 ng/ml, at or above about 380 ng/ml, at or above about 400 ng/ml, at or above about 420 ng/ml, at or above about 440 ng/ml, at or above about 460 ng/ml, at or above about 480 ng/ml, at or above about 500 ng/ml, at or above about 520 ng/ml or at or above about 540 ng/ml.

A method as described above may further include detecting the amount of the 76 amino acid N-terminal fragment of Brain natriuretic peptide (NT-pro BNP) in the sample. In some embodiments a method according to the invention may include detecting the amount of Brain natriuretic peptide (BNP) in the sample. In some embodiments a method as described above may include detecting the amount of troponin T in the sample. In some embodiments a method as described above may include detecting the amount of troponin I in the sample. In one embodiment a respective method includes detecting the amount of NT-pro BNP and troponin T in the sample. In one embodiment a respective method includes detecting the amount of NT-pro BNP and troponin I in the sample. Further, the amount(s) of NT-pro BNP, troponin T and/or troponin I, as applicable, in the sample is/are compared to a threshold value. An increased amount of NT-pro BNP, relative to the threshold value, is further indicative of a complication of obstructive pulmonary disease. Likewise, an increased amount of troponin T and/or troponin I, relative to the threshold value, is further indicative of a complication of obstructive pulmonary disease. The above explanations on techniques of detection, modes of measurement and the establishment and use of one or more threshold values apply mutatis mutandis to determining levels of NT-pro BNP, troponin T and troponin I.

Brain natriuretic peptide, also known as Natriuretic peptide B or B-type natriuretic peptide may be any respective variant or isoform of the respective species, e.g. human. The protein may for example be the human protein of the Swissprot/Uniprot accession number P16860 (version 124 as of 22 February 2012). Troponin T may be any respective variant or isoform of the respective species, e.g. human. The protein may for example be the human cardiac isoform of the protein, which has the Swissprot/Uniprot accession number P45379 (version 123 as of 22 February 2012). Troponin I may be any respective variant or isoform of the respective species, such as human. The protein may for example be the human cardiac isoform of the protein, which has the Swissprot/Uniprot accession number P19429 (version 124 as of 22 February 2012).

Accordingly, a method that includes e.g. determining the amount of proSP-B and NT-pro BNP, a method that includes determining the amount of proSP-B and troponin T, a method that includes determining the amount of proSP-B and troponin I, or a method that includes determining the amount of proSP-B, NT-pro BNP and troponin T and/or troponin I is in some embodiments a methods where a complication of obstructive pulmonary disease is being diagnosed. In some embodiments of such a method the sample is a sample from a human that does not have a history of heart failure.

In some embodiments of a method according to the invention, in which a level of NT-pro BNP and/ or a level of Troponin T is determined, the sample originates, e.g. has been taken, from a subject that/who is known not to suffer from impaired or reduced renal function. Reduced function of the kidneys can result in an accumulation of NT-pro BNP and/ or Troponin T, thereby causing elevated levels thereof. In some embodiments of a method according to the invention, in which a level of proSP-B is determined, the sample originates, e.g. has been taken, from a subject that/who is known not to have an impaired or reduced renal function.

Where the sample is a sample from a human that does not have a history of heart failure, an increased amount of NT-pro BNP, relative to the threshold value, is further indicative of a complication of obstructive pulmonary disease. Likewise, an increased amount of troponin T and/or troponin I, relative to the threshold value, is further indicative of a complication of obstructive pulmonary disease. A value above about 220 pg/ml NT-pro BNP, including above about 250 pg/ml, above about 270 pg/ml, above about 300 pg/ml, above about 320 pg/ml, above about 350 pg/ml, above about 370 pg/ml or above about 400 pg/ml proSP-B may indicate a complication of obstructive pulmonary disease. A value above about 3 pg/ml troponin T, including above about 5 pg/ml, above about 7 pg/ml, above about 8 pg/ml, above about 9 pg/ml, above about 10 pg/ml, above about 11 pg/ml, or above about 12 pg/ml, troponin T may indicate a complication of obstructive pulmonary disease.

Where in a method according to the invention the sample is a sample from a human that does have a history of heart failure, a threshold value is advantageously based on samples taken from the subject previously, namely at a point in time where the subject has been known not to suffer from elevated severity of obstructive pulmonary disease. In some embodiments the sample is from a human that has a history of heart failure and the human is suspected to suffer from a state of obstructive pulmonary disease of increased severity. An increased amount of proSP-B, relative to the threshold value, indicates that the subject is suffering from exacerbated obstructive pulmonary disease. An increased amount of NT-pro BNP, relative to the threshold value, is further indicative of a complication of obstructive pulmonary disease.

Heart failure is a common syndrome well known in the art. Heart failure may be right and/or left sided. In this regard, left ventricular dysfunction is the primary cause of right ventricular failure. A subject with left heart failure generally has symptoms of low cardiac output and elevated pulmonary venous pressure, typically associated with dyspnoea. Shortness of breath is the most common complaint of subjects suffering from left ventricular failure, often accompanied by a chronic cough that can easily be overlooked. Fatigue and exercise intolerance are also common symptoms reported by patients with left heart failure. As can for instance be seen from the similarity of symptoms to symptoms of obstructive pulmonary disease, early stages of heart failure may remain undetected as heart failure, i.e. be left undiagnosed - in particular if no history of heart failure is known. Furthermore, symptoms of mild stages of heart failure such as Class I and Class II according to the New York Heart Association (NYHA) classification, e.g. fatigue, may be misinterpreted as spring tiredness or attributed to a different condition such as depression or a malign disease. In some embodiments where a method according to the present invention is applied, it should thus be taken into consideration that an inappropriate threshold value may have been selected. As a safeguard it may under such circumstances be advantageous to use samples taken from the same subject at an earlier point in time (supra) as a reference.

A complication of obstructive pulmonary disease may be any known complication of COPD. Typically the complication is a systemic infection. A respective complication may in some embodiments be an acute exacerbation of COPD, which becomes apparent as an abrupt increase of symptoms. The response of the immune system to systemic infection is known as the systemic inflammatory response syndrome (SIRS). In some embodiments the complication is sepsis. Both SIRS and sepsis can be a response to any class of microorganism, including, but not limited to, bacteria, both gram-negative and gram-positive bacteria, fungi and viruses. It is understood that in a respective state it is to a large extent the inflammatory response itself that can lead to tissue damage and death, rather than the infection. The syndrome is accompanied by activation of several types of cells, including lymphocytes, macrophages, dendritic cells, endothelial cells, platelets, and neutrophils. SIRS is an excessive inflammatory response that can be characterized by a release of numerous pro-inflammatory mediators, often a high production of cytokines such as TNF-alpha, IL-1, IL-6, IL-8, and PAF is observed. Dilation of blood vessels, followed by massive adhesion of neutrophils to capillary walls, activation of neutrophils, which leads to release of proteases and oxygen radicals can cause damage to the capillary walls. Further, prolonged vasodilation and increased capillary permeability can result in the entry of plasma into tissue. Prolonged vasodilation further results in decreased vascular resistance that, in turn, causes hypotension. In addition, activation of the blood coagulation pathway and concurrent down-regulation of anticoagulation mechanisms cause clots to form within the blood vessels throughout the body (disseminated intravascular coagulation). The increased capillary permeability and injury to capillaries in the alveoli of the lungs can result in acute inflammation, pulmonary edema, and loss of gas exchange. This is called acute respiratory distress syndrome (ARDS). The above cascade of events can result in irreversible septic shock, multiple system organ failure, and death.

For human individuals at the age of 16 and above SIRS without organ complications is diagnosed if two or more of the following criteria are given: the body temperature is less than 36 °C or above 38 °C, tachycardia with a heart rate greater than about 90 beats per minute, tachypnea with greater than 20 breaths per minute or an arterial partial pressure of carbon dioxide below 4.3 kPa, leucocyte counts are below 4000 cells/mm³ (4 x 10⁹ cells/L) or greater than 12,000 cells/mm³ (12 x 10⁹ cells/L) or greater than 10% immature neutrophils (band forms) are present.

Rapid identification of SIRS and in particular sepsis and its causative microorganisms is the basis for successful treatment. Unfortunately, both may be difficult with the current clinically available methods. Therefore those skilled in the art will appreciate that a method according to the invention in which levels of proSP-B and one or more of NT-pro BNP, troponin T and troponin I are determined, provides a fast and reliable means of diagnosing SIRS.

Sepsis as well as severe SIRS are known to involve cardiac impairment. Further, NT-proBNP, BNP, Troponin T and Troponin I are known to be elevated in patients with cardiac injury and heart failure. Without being bound by theory, the inventors' finding that increased levels of these proteins indicate a complication of obstructive pulmonary disease such as SIRS may point to cardiac involvement in earlier stages of SIRS than severe SIRS.

With regard to setting a threshold value for NT-pro BNP, BNP, troponin T, and troponin I, the above explanations on the use of control sample values and calculations applies mutatis mutandis. Thus with a plurality of control samples compared to a value in a sample of NT-pro BNP, BNP, troponin T, and/or troponin I, a p value of 0.05, 0.02, 0.01 or lower may be taken to indicate a difference, or a value above e.g. the 90^{th} percentile, the 92^{nd} percentile, the 93^{rd} percentile, the 94^{th} percentile, the 95^{th} percentile, the 96^{th} percentile, the 97^{th} percentile, the 98^{th} percentile or the 99^{th} percentile, as well as e.g. a two-fold or more, including 3 fold or more, such as at least about 5 to about 10 fold value of the additional marker in the sample may be regarded as an elevated level.

As indicated above, the invention also provides a use of a binding agent for assessing the severity of obstructive pulmonary disease in a subject. The above said with regard to a method of determining the severity of obstructive pulmonary disease in a subject applies mutatis mutandis to a respective use. In such a use the amount of proSP-B determined in the sample may be compared to a threshold value, which may in some embodiments be a predetermined threshold value. As explained above, in some embodiments the threshold value is based on the amount of proSP-B in a control sample (supra), which may for instance be from a different subject known not to suffer from obstructive pulmonary disease, from a different subject known not to suffer from exacerbated obstructive pulmonary disease or from the same subject at a time where the subject was known not to have an increased severity of obstructive pulmonary disease. A threshold value may in some embodiments be a collection of data of a plurality of control samples (supra). As explained above, where a p value of a plurality of measurements, has been determined, a p value of 0.05, 0.02, 0.01 or lower may be taken to indicate a difference. In some embodiments a deviation of a value of a test sample relative to a value of a control sample of about 2 fold or more, including 3 fold or more, such as at least about 5 to about 10 fold or even more may be regarded as a significant increase. As explained above, in some embodiments a certain percentile of a collection of data may be used as a threshold value, for example a level of proSP-B above the 90^{th} percentile, above the 92^{nd} percentile, above the 93^{rd} percentile, above the 94^{th} percentile, above the 95^{th} percentile, above the 96^{th} percentile, above the 97^{th} percentile, above the 98^{th} percentile or above the 99^{th} percentile may be regarded as increased or elevated. In some embodiments the threshold value may be based on the value of standard deviation or relative standard deviation of a plurality of control measurements and/or control samples, standard deviation or relative standard deviation for instance being multiplied with a factor, which may be larger or smaller than 1, such as 1.2, 1.5 or 2. In some embodiments an average of a selected number, such as 2, 3, 4, 5 or more of the highest proSP-B levels determined, for instance over time, may be used as a predetermined threshold value.

In some embodiments of a use according to the invention the determined level of proSP-B is regarded as increased where the obtained value is about 1.5 standard deviations higher or lower, including about two, about three, about four or more standard deviations higher or lower than the average value determined in a control sample. In some embodiments the determined amount of proSP-B is regarded as different where the obtained value is about 1.2 times or more higher or lower, including about 1.5 times, about two fold, about 2.5-fold, about three fold, about 3.5 fold, about 4-fold, about 5-fold or more higher or lower than the protein level determined in a control sample. In some embodiments the determined level of proSP-B is regarded as increased where the obtained value is about 0.8-fold or less, including about 70 %, about 60 %, about 50 %, about 40 %, about 30 %, about 25 %, about 20 % or lower than the of proSP-B level determined in a control sample.

In some embodiments in addition to detecting the amount of proSP-B, or an effective portion thereof, the amount of NT-pro BNP (supra), the amount of troponin T or troponin I, and the amount of C-reactive protein (CRP) is detected in the sample. C-reactive protein, also known as PTX1, may be any respective variant or isoform of the respective species, e.g. human. The protein may for example be the human protein of the Swissprot/Uniprot accession number P02741 (version 152 as of 22 February 2012).

A respective method is a method of stratifying a subject for obstructive pulmonary disease therapy. The terms "stratifying" and "stratification" as used herein indicate in one aspect that individuals are assigned to groups with similar characteristics such as at a level of obstructive pulmonary disease. As an illustrative example, individuals may be stratified into risk categories, for example whether the individual is or is not at a disease state where potential life threatening changes are to be taken into account. The terms "stratifying" and "stratification" as used herein indicate in another aspect that an individual is assigned to a certain group according to characteristics matching the respective group such as a corresponding level, including risk level of obstructive pulmonary disease. The groups may be, for example, for testing, prescribing, suspending or abandoning any one or more of a drug, surgery, diet, exercise, or intervention. Accordingly, in some embodiments of a method or use according to the invention a subject may be stratified into a subgroup of a clinical trial of a therapy. As explained in the forgoing, in the context of the present invention proSP-B, NT-pro BNP, BNP, troponin T and CRP may be used for obstructive pulmonary disease stratification.

The terms "stratifying" and "stratification" according to the invention generally include identifying subjects that require an alteration of their current or future therapy. The term includes assessing, e.g. determining, which therapy a subject suffering from obstructive pulmonary disease is in need of. Hence, in the context of the present invention stratification may be based on the health risk involved with a subject's state of obstructive pulmonary disease. A method or use according to the invention may also serve in stratifying the risk of any COPD related condition for a subject. A method of stratifying a subject for therapy of obstructive pulmonary disease according to the invention includes detecting the amount of proSP-B, or an effective portion thereof, NT-pro BNP, troponin T, and CRP as described above. As explained above, in some embodiments on a general basis a proSP-B binding partner can be advantageously used to screen risk patients which have higher severity of COPD. As will be apparent from the above, terms "stratifying" and "stratification" include the staging of subjects. The stage of obstructive pulmonary disease in terms of disease severity and/or the degree of exacerbation can be assessed using a method according to the invention, as well as the degree of complication of obstructive pulmonary disease.

The term "stratification" according to the invention generally includes identifying subjects that do not require hospitalisation and immediate antibiotic therapy, identifying subjects that may be in need of hospitalisation and immediate antibiotic therapy, as well as identifying subjects that are in need of intense surveillance and antibiotic therapy. The term includes assessing, e.g. determining, which therapy a subject suffering from obstructive pulmonary disease.

In this regard the use of biomarkers for stratification of patients is a well established procedure in the art. This procedure includes or consists of linking one or more patient subpopulations, characterized by a certain feature, in the context of the present invention the level of particular proteins, to a particular treatment. The general aim of stratification is to match patients with therapies that are more likely to be effective and safe. In a more general context stratifying patients may include evaluation of patient history and physical assessment, combined with laboratory tests on the basis of a method of the present invention, and clinical observation. It is understood that stratifying patients is only feasible if multiple treatment options with heterogeneous responses for the disease exist. In the context of the present invention therapy of obstructive pulmonary disease and patient surveillance may be adjusted. A general overview of patient stratification and stratified medicine has been given by Trusheim, M.R., et al., Nature Reviews Drug Discovery (2007) 6, 4, 287-293.

A method of stratifying a subject for obstructive pulmonary disease therapy according to the invention includes detecting the amount of pro-SP-B, or an effective portion thereof, as described above. The amount of NT-pro BNP or BNP, troponin T or troponin I, and CRP may be determined in a corresponding manner, using the above described techniques and methods. Levels below high levels of each of NT-pro BNP or BNP, troponin T or troponin I, proSP-B and CRP in the sample indicate that the subject is not in need of intense surveillance and antibiotic therapy. Low levels of each of pro-SP-B, NT-pro BNP or BNP, troponin T or troponin I, and CRP indicate that a subject does not require intense surveillance and antibiotic therapy. Likewise, high levels of pro-SP-B, low levels of NT-pro BNP or BNP, low levels of troponin T or troponin I, and intermediate levels of CRP indicate that a subject does not require intense surveillance and antibiotic therapy. High levels of pro-SP-B, high or very high levels of NT-pro BNP or BNP, high or very high levels of troponin T or troponin I, and high levels of CRP indicate that a subject may require at least one of intense surveillance and antibiotic therapy. High levels of pro-SP-B, very high levels of NT-pro BNP, very high levels oftroponin T or troponin I, and high levels of CRP indicate that the subject's condition needs to be monitored and that the subject is in need of therapy with antibiotics, to which the subject shows no resistance. Very high levels of each of NT-pro BNP or BNP, and troponin T or troponin I, and high levels of proSP-B and CRP in the sample relative to the corresponding threshold value, indicate that the subject is in need of intense surveillance and/or antibiotic therapy.

A low level of NT-pro BNP is typically a value below about 125 pg/ml, such as below about 115 pg/ml, below about 100 pg/ml or below about 90 pg/ml. A medium level of NT-pro BNP is typically a value in the range from about 125 pg/ml to about 500 pg/ml, such as a value in the range from about 130 pg/ml to about 500 pg/ml, from about 150 pg/ml to about 480 pg/ml or in the range from about 170 pg/ml to about 450 pg/ml. A high level of NT-pro BNP is typically a value in the range from about 500 pg/ml to about 1500 pg/ml, such as a value in the range from about 520 pg/ml to about 1450 pg/ml, from about 550 pg/ml to about 1400 pg/ml or in the range from about 580 pg/ml to about 1350 pg/ml. A very high level of NT-pro BNP is typically a value above about 1500 pg/ml, such as above about 1550 pg/ml or above about 1600 pg/ml. A low level of Troponin T is typically a value below about 3 pg/ml, such as below about 2.8 pg/ml or below about 2.5 pg/ml. A medium level of Troponin T is typically a value in the range from about 3 pg/ml to about 7 pg/ml, such as a value in the range from about 3.2 pg/ml to about 6.8 pg/ml or a value in the range from about 3.5 pg/ml to about 6.5 pg/ml. A high level of Troponin T is typically a value in the range from about 7 pg/ml to about 20 pg/ml, such as a value in the range from about 7.2 pg/ml to about 19.8 pg/ml, in the range from about 7.5 pg/ml to about 19.5 pg/ml, in the range from about 8 pg/ml to about 19 pg/ml or in the range from about 8.5 pg/ml to about 18.5 pg/ml. A very high level of Troponin T is typically a value above about 20 pg/ml, such as above about 20.5 pg/ml, above about 21 pg/ml, above about 22 pg/ml, or above about 25 pg/ml.

A low level of pro SP-B is typically a value below about 35 ng/ml, such as below about 34 ng/ml, below about 32 ng/ml or below about 30 ng/ml. A medium level of pro SP-B is typically a value in the range from about 35 ng/ml to about 70 ng/ml, such as a value in the range from about 36 ng/ml to about 68 ng/ml, from about 38 ng/ml to about 65 ng/ml or in the range from about 40 ng/ml to about 63 ng/ml. A high level of pro SP-B is typically a value in the range from about 70 ng/ml to about 150 ng/ml, such as a value in the range from about 72 ng/ml to about 145 ng/ml, from about 75 ng/ml to about 140 ng/ml or in the range from about 77 ng/ml to about 135 ng/ml. A very high level of pro SP-B is typically a value above about 150 ng/ml, such as above about 155 ng/ml or above about 160 ng/ml. A low level of CRP is typically a value below about 3 mg/ml, such as below about 2.8 mg/l or below about 2.5 mg/l. A medium level of CRP is typically a value in the range from about 3 to about 15 mg/l, such as a value in the range from about 3.2 mg/l to about 14.5 mg/l or from about 3.5 mg/l to about 14 mg/l. A high level of CRP is typically a value in the range from about 15 mg/l to about 40 mg/l, such as a value in the range from about 15.5 mg/l to about 38 mg/l, from about 15 mg/l to about 35 mg/l or in the range from about 15.5 mg/l to about 32 mg/l. A very high level of CRP is typically a value above about 40 mg/l, such as above about 42 mg/l or above about 45 mg/l.

In the method of stratifying a subject for obstructive pulmonary disease therapy the amount of pro-SP-B, NT-pro BNP or BNP, troponin T or troponin I, and CRP determined in the sample may be compared to a threshold value. Such a threshold value may in some embodiments be a predetermined threshold value with regard to the respective marker. In some embodiments the threshold value is based the amount of proSP-B in a control sample. A respective control sample may have any condition that varies from the sample main measurement itself. Such a control sample may be a sample of, or including, the corresponding body fluid as the sample from the subject. A control sample may for example be a sample, such as a blood sample, a plasma sample or a serum sample, of a subject known not to suffer from obstructive pulmonary disease. A control sample may also be from a subject known not to suffer from exacerbated obstructive pulmonary disease. In some embodiments a respective control sample is from a subject that is age-matched. In some embodiments a respective control sample is from a subject that is known not to have a confounding disease, in some embodiments from a subject known not to have a disease.

In some embodiments a threshold value is a collection of data of a plurality of control samples (supra). As explained above, the threshold value may be set to be a significant difference between the control and the sample from the subject of interest. For instance a p value of 0.05, 0.02, 0.01 or lower may be taken to indicate a difference (supra). In some embodiments a significant increase is a deviation of a value of a test sample relative to a value of a control sample of about 2 fold or more, including 3 fold or more, such as at least about 5 to about 10 fold or even more.

The above said on taking and the origin of a control value, which may consist of or be based on a plurality of individual values, that may be used applies mutatis mutandis to a method of stratifying a subject for obstructive pulmonary disease therapy. In some embodiments a percentile such as a level of a selected biomarker may be regarded as increased or elevated if it is above the 90^{th} percentile, above the 92^{nd} percentile, above the 93^{rd} percentile, above the 94^{th} percentile, above the 95^{th} percentile, above the 96^{th} percentile, above the 97^{th} percentile, above the 98^{th} percentile or above the 99^{th} percentile. In some embodiments the threshold value may be based on the value of standard deviation or relative standard deviation of a plurality of control measurements and/or control samples, standard deviation or relative standard deviation for instance being multiplied with a factor, which may be larger or smaller than 1, such as 1.2, 1.5 or 2. In some embodiments an average of a selected number, such as 2, 3, 4, 5 or more of the highest levels of a selected biomarker determined may be used as a predetermined threshold value.

In some embodiments the average value of data from patients known to suffer from COPD without exacerbation may be determined and used to determine a threshold value. As an illustrative example, the value of standard deviation or relative standard deviation, in some embodiments multiplied with a factor, which may be larger or smaller than 1. In some embodiments a multiple of standard deviation or relative standard deviation, such as 1.2, 1.5 or 2 times the standard deviation, may be added to the average value determined. In some embodiments establishing a predetermined threshold value includes collecting from patients known to suffer from COPD without exacerbation over a period of time, such as a plurality of months. Average proSP-B levels, standard deviation, and relative standard deviation at given times may be calculated for the patients to determine a range of proSP-B levels associated with COPD without exacerbation thereof. In some embodiments an average of a selected number, such as 2, 3, 4, 5 or more of the highest proSP-B levels determined over time may be used as a predetermined threshold value. In some embodiments the average value of all data collected at different time points may be determined and used to determine a threshold value, for instance by adding standard deviation, relative standard deviation or e.g. a multiple thereof (supra). When test result from a patient to be evaluated is collected, it will be compared to the threshold value. Statistical differences between the test result and the threshold value will be determined to identify significant variances in between.

The amount of proSP-B determined in or from a sample of a subject can be compared to a single control sample or a plurality of control samples, such as a sample from a control subject, in any suitable manner. As an illustrative example, the level of proSP-B in a control sample can be characterized by an average (mean) value coupled with a standard deviation value, for example at a given time point. In some embodiments the level of proSP-B in a subject may be considered increased when it is one standard deviation or more higher than the average value of the corresponding expression level determined in one or more control samples. In some embodiments the determined level of proSP-B is regarded as increased where the obtained value is about 1.5 standard deviations higher or lower, including about two, about three, about four or more standard deviations higher or lower than the average value determined in a control sample. In some embodiments the determined amount of proSP-B is regarded as different where the obtained value is about 1.2 times or more higher or lower, including about 1.5 times, about two fold, about 2.5-fold, about three fold, about 3.5 fold, about 4-fold, about 5-fold or more higher or lower than the protein level determined in a control sample. In some embodiments the determined level of proSP-B is regarded as increased where the obtained value is about 0.8-fold or less, including about 70 %, about 60 %, about 50 %, about 40 %, about 30 %, about 25 %, about 20 % or lower than the of proSP-B level determined in a control sample.

If for example a level of proSP-B is detected that is above a, e.g. predetermined, threshold value, this may indicate an increase in severity of obstructive pulmonary disease. A level of proSP-B above a threshold value may indicate a condition where the subject is suffering from severe exacerbated COPD. If a level of proSP-B is detected that is below a predetermined threshold value, this may indicate that no increase in severity of obstructive pulmonary disease has occurred.

As explained above, in embodiments where the sample is a sample from a human that has a history of heart failure, a threshold value is advantageously based on samples taken from the subject at an earlier point in time, namely at a point in time where the subject has been known not to suffer from obstructive pulmonary disease, or at least a point in time where the subject has been known not to suffer from elevated severity of obstructive pulmonary disease.

Provided herein is also a combination of a plurality of antibodies, which may include one or more immunoglobulins and/or one or more proteinaceous binding molecules. The combination includes a first immunoglobulin or a proteinaceous binding molecule that is specific for proSP-B. The combination further includes a second immunoglobulin or proteinaceous binding molecule. The second immunoglobulin or proteinaceous binding molecule is in some embodiments specific for NT-pro BNP or for BNP. In some embodiments the second immunoglobulin or proteinaceous binding molecule is specific for troponin T or for troponin I. The second immunoglobulin or proteinaceous binding molecule is in some embodiments specific for CRP. In one embodiment the combination includes an immunoglobulin or a proteinaceous binding molecule specific for proSP-B, an immunoglobulin or proteinaceous binding molecule specific for NT-pro BNP or for BNP, an immunoglobulin or proteinaceous binding molecule specific for troponin T or for troponin I, and an immunoglobulin or proteinaceous binding molecule is in some embodiments specific for CRP.

In some embodiments the combination is provided in the form of a kit. The kit includes a first container that includes the first immunoglobulin or a proteinaceous binding molecule with immunoglobulin-like functions, which is specific for proSP-B. The kit further includes a second container that includes the second immunoglobulin or a proteinaceous binding molecule. In one embodiment the kit includes a container that includes an immunoglobulin or a proteinaceous binding molecule with immunoglobulin-like functions, which is specific for proSP-B. The kit further includes a container that includes an immunoglobulin or a proteinaceous binding molecule with immunoglobulin-like functions, which is specific for NT-pro BNP or for BNP. The kit also includes a container that includes an immunoglobulin or a proteinaceous binding molecule with immunoglobulin-like functions specific for troponin T or for troponin I. Furthermore the kit includes a container that includes an immunoglobulin or a proteinaceous binding molecule with immunoglobulin-like functions specific for CRP. The kit may further include instructions and/or imprint indicating that a patient is to be assessed and/or stratified by a method described herein; and/or instructions regarding how to carry out a method as defined herein. It may also include positive and/or negative controls which allow a comparison to the control. The kit may enable the assessment of a patient's treatment progress and the presence of exacerbated obstructive pulmonary disease. A respective kit may be used to carry out a method according to the present invention. It may include one or more devices for accommodating the above components before, while carrying out a method of the invention, and thereafter.

In order that the invention may be readily understood and put into practical effect, particular embodiments will now be described by way of the following non-limiting examples.

### EXAMPLES

### Patients and Methods:

### 1. Patients:

A total of 71 patients with exacerbated COPD were included into the study, all patients presented with increased dyspnoea, purulent sputum and fever of at least 38.5 degrees Celsius. Chest X ray revealed to radiographic signs of pneumonia. Thus the final diagnosis was exacerbated COPD.

Because COPD and cardiovascular diseases share similar risk factors 158 patients with documented cardiovascular disease who had no concomitant lung disorder and who had no evidence of clinical heart failure were also included into the study. Moreover 31 patients with clinical heart failure and without concomitant lung disorder were also tested.

### 2. Methods:

NT-pro BNP, troponin T and CRP were tested using commercially available assays (Roche Diagnostics) and proSP-B were determined using prototype Elecsys tests. The lower detection limit in the troponin T test used was 1 pg/ml. All other tests were done using standard laboratory tests and established evaluation criteria. Imaging was done as needed using routine methods.

### 2.1 Assay for measurement of proSP-B

### 2.1.1 Antibodies used

The proSP-B assay uses a mouse monoclonal anti-proSP-B (N-terminus) antibody as a capture and a mouse monoclonal anti-proSP-B (C-terminus) antibody as a detection reagent. The assay principle is a sandwich format. The antibody to the N-terminal pro-sequence (clone 1.14.133) binds to an epitope included in the peptide sequence ranging from amino acid positions 160 to 169 of proSP-B. The antibody to the C-terminal pro-sequence (clone 1.7.41) binds to an epitope included in the peptide sequence ranging from amino acid positions 323 to 334 of proSP-B. Detection is based on an electrochemiluminescence immunoassay (ECLIA), using a Tris(bipyridyl)-ruthenium(II) complex as a label.

### 2.1.2 Assay procedure

The biotinylated capture antibody (80 µl), the ruthenium-labeled detection antibody (80 µl), and sample or standard material (10 µl) are incubated in homogeneous phase for 9 min at 37 °C. Concentrations in the stock solution were 1.7 µg/ml for the biotinylated capture antibody and 1.2 µg/ml for the ruthenylated detection antibody, respectively. After the first nine minutes 30 µl of Streptavidin-coated beads are added, and binding of the immune complexes formed to the microparticles is allowed to take place during a further incubation of 9 minutes. After the second incubation, the reaction mixture is transferred into the measuring cell, where beads are captured to the electrode surface by a magnet. The measuring cell is washed to remove unbound label and filled with detection buffer containing Tris-propylamine. After applying voltage to the electrode, the emitted chemiluminescence light is detected by a photomultiplier. Results are determined via a 2-point calibration curve. The corresponding concentration for proSP-B is given in pg/ml.

### 2.2 Assay for measurement of C-terminal proSP-B

### 2.2.1 Antibodies used

The proSP-B assay uses a first mouse monoclonal anti-proSP-B (C-terminus) antibody as a capture reagent and a second mouse monoclonal anti proSP-B (C-terminus) antibody as a detection reagent. The assay principle is a sandwich format. The antibody to the first C-terminal pro-sequence (clone 1.7.41) binds to an epitope included in the peptide sequence ranging from amino acid positions 323 to 334 of proSP-B. The antibody to the second C-terminal pro-sequence (clone 1.3.9) binds to an epitope included in the peptide sequence ranging from amino acid positions 285 to 294 of proSP-B. Detection is based on an electrochemiluminescence immunoassay (ECLIA), using a Tris(bipyridyI)-ruthenium(II) complex as a label.

The biotinylated capture antibody (MAB 1.7.41) (80 µl), the ruthenium-labeled detection antibody (MAB 1.3.9) (80 µl), and sample or standard material (10 µl) are incubated in homogeneous phase for 9 min at 37°C. Concentrations in the stock solution were 1.5 µg/ml for the biotinylated capture antibody and 1.0 µg/ml for the ruthenylated detection antibody, respectively. After the first nine minutes 30 µl of Streptavidin-coated beads are added, and binding of the immune complexes formed to the microparticles takes place during a further 9-min incubation. After the second incubation, the reaction mixture is transferred into the measuring cell, where beads are captured to the electrode surface by a magnet. The measuring cell is washed to remove unbound label and filled with detection buffer containing Tris-propylamine. After applying voltage to the electrode, the emitted chemiluminescence light is detected by a photomultiplier. Results are determined via a 2-point calibration curve. The corresponding concentration for C-terminal proSP-B is given in pg/ml.

### 3. Analysis of clinical samples

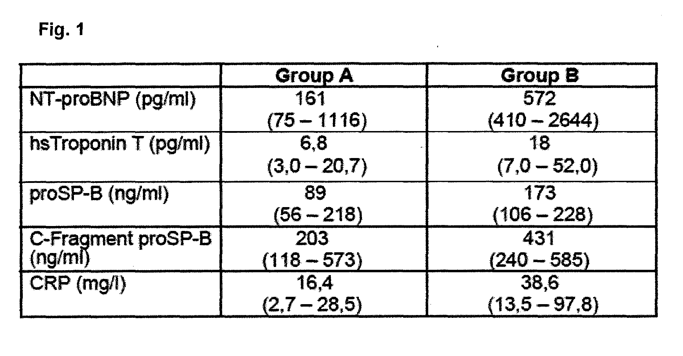

In a first step patients were selected as to whether they met SIRS (systemic inflammatory response syndrome) criteria which is defined by two or more of the following criteria: fever of more than 38 degrees celsius or hypothermia (below 36 degrees), tachypnoe, tachycardia and leucocytosis (above 12.000) or leucopenia (below 4000). Fever and absent leucocytosis and leucopenia was present in 32 patients (Group B) and absent in 32 patients (Group A) of a total of 65 patients in whom all information available. Data for both groups are summarized in Fig. 1.

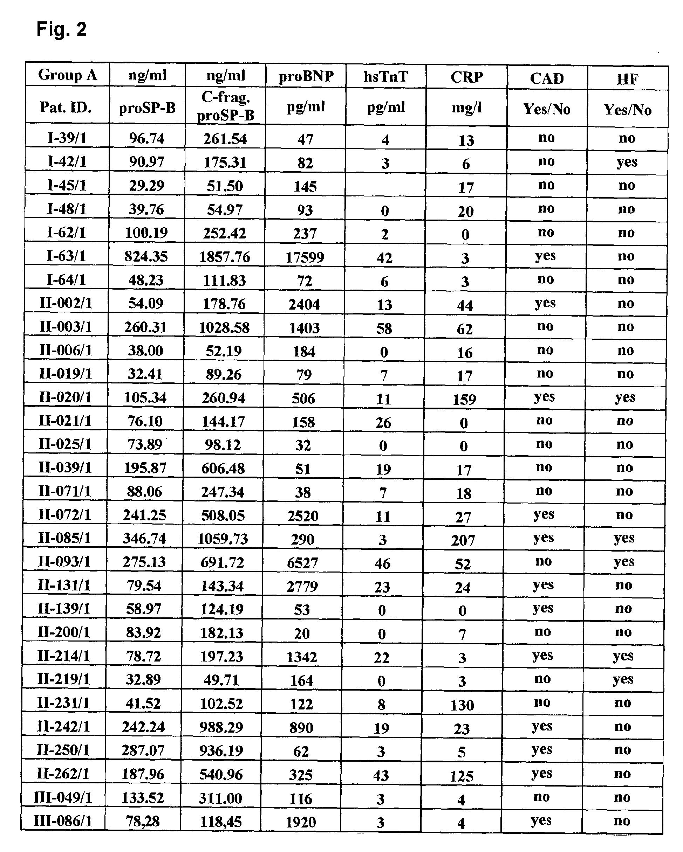

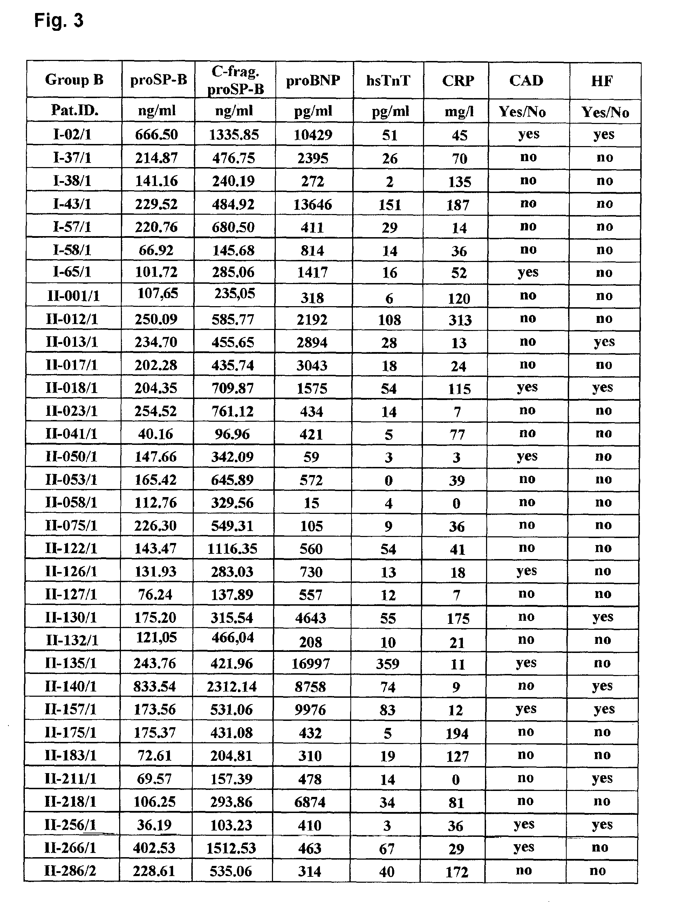

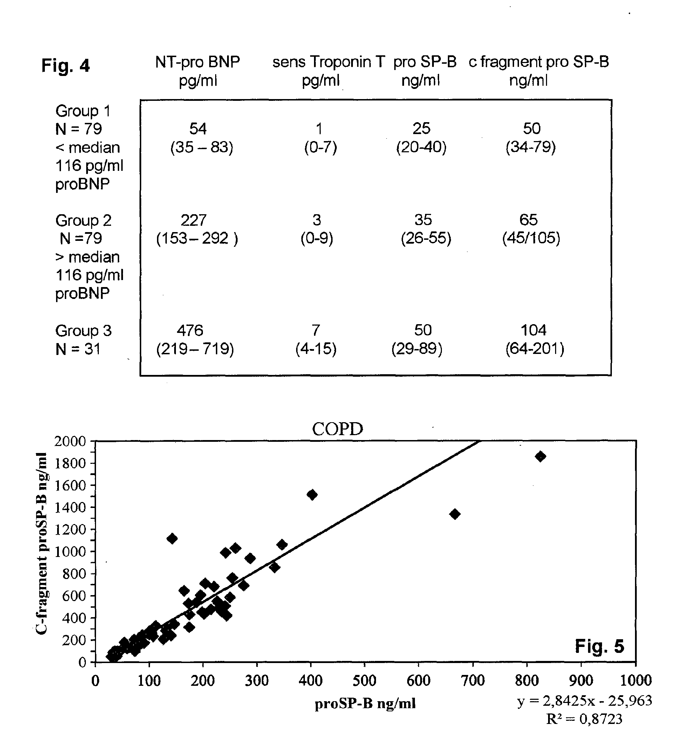

As can be seen from the Table in Fig. 1, exacerbated COPD patients with SIRS criteria had higher NT-pro BNP, troponin T, CRP, and pro SP-B values. Individual cases of Group A and Group B patients for the markers listed are given in Fig. 2 and Fig. 3, respectively. Data obtained from the control population are shown in Fig. 4. As can be taken from Fig. 4, patients with and without cardiovascular disease and without a history of heart failure exhibit pro SP-B values below 35 (26-55) pg/ml and c-fragment pro SP-B levels below 65 (45/105) pg/ml indicating that levels above these reference values indicate pulmonary involvement associated with COPD, in case of pre-existing clinical heart failure pro SP-B values before exacerbation are required for decision making. Similar considerations apply for NT-pro BNP and troponin T.

### 4. Conclusion

As becomes clear from the results of the study, proSP-B and C-fragment proSP-B recognize involvement of type 2 pneumocytes in exacerbated COPD and thus a pulmonary complication not recognized by chest X ray. Pro-SP-B was shown to be higher in patients meeting the SIRS criteria than those not meeting these SIRS criteria and thus might represent an early sign of an acute lung injury, which may be subclinical or clinical, not recognized by other methods. A shown in the Figure below, proSP-B and C-fragment proSP-B provided identical information.

The source of NT-pro BNP and sensitive Troponin T is uncertain. Possible sources include concomitant coronary artery disease and heart failure, however there was no good correlation with left ventricular function as assessed by echocardiography, similarly advanced COPD might result in secondary pulmonary hypertension, however there was no good correlation to right ventricular function. The fact that patients with signs of SIRS have higher NT-pro BNP and troponin T concentrations point to the fact that NT-pro BNP and troponin T represent early markers of systemic inflammation, specifically in those in whom heart failure was absent in the past.

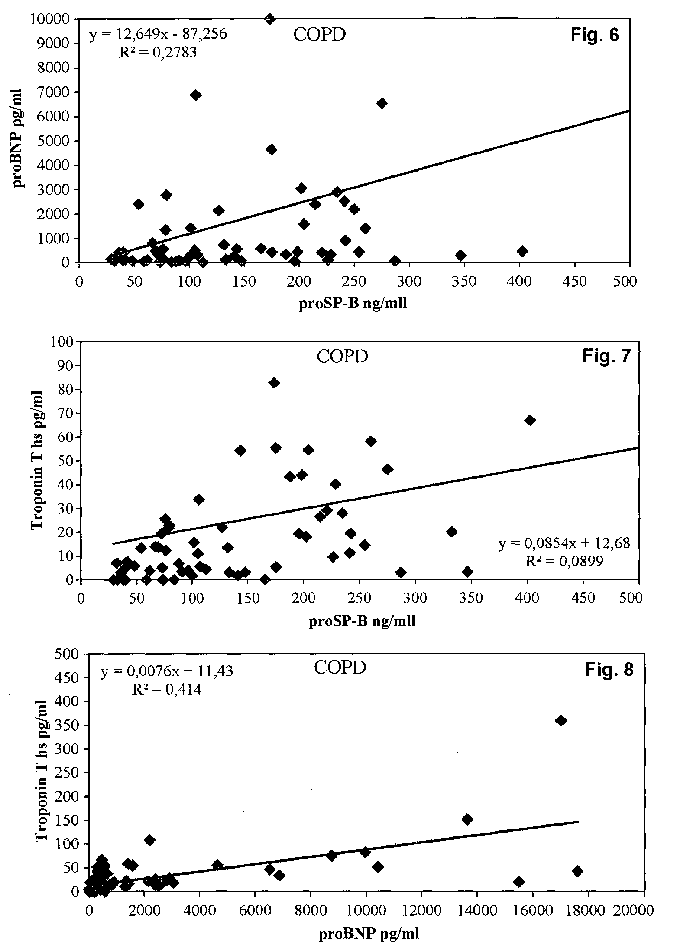

Figures 6 to 8 represent a correlation of pro SP-B to NT-proBNP and sensitive Troponin T. As becomes clear from these figures, there is a correlation between proSP-B and both markers, however this correlation is not very strong, indicating that each of these markers is independent.

As outlined before classification of exacerbated COPD is difficult based on clinical signs and symptoms so is recovery of pathogens and decision to treat.

In view of the present invention evaluation of pulmonary damage can be added to the assessment of COPD where SIRS criteria beyond leucocytosis and leucopenia are of little help. Data obtained in this study indicate that elevated NT-proBNP and troponin T levels are frequently not related to underlying cardiac diseases such as CAD or pulmonary arterial hypertension but rather the result of a COPD complication. In this context, increase of proSP-B needs to be considered as involvement of small airways and alveoli possibly as a sign of an early acute lung injury and may predispose to secondary pneumonia.

This results in the algorithm of the following Table 1.

**Table 1: Stratification of individuals**

| **2. Risk** | **NT-proBNP** | **hsTroponin T** | **proSP-B** | **CRP** |
|---|---|---|---|---|
| Low | low | low | low | low |
| Intermediate | low | low | high | intermediate |
| High | high | high | high | high |
| Very high | Very high | Very high | high | high |

Whereas low and intermediate risk patients do not require hospitalisation and immediate antibiotic therapy, this needs to be carefully considered in high and very high risk patients. Very high risk patients require intense surveillance, broad spectrum antibiotic therapy and resistance testing.

The listing or discussion of a previously published document in this specification should not necessarily be taken as an acknowledgement that the document is part of the state of the art or is common general knowledge.

The invention illustratively described herein may suitably be practiced in the absence of any element or elements, limitation or limitations, not specifically disclosed herein. Thus, for example, the terms "comprising", "including," containing", etc. shall be read expansively and without limitation. Singular forms such as "a", "an" or "the" include plural references unless the context clearly indicates otherwise. Unless otherwise indicated, the term "at least" preceding a series of elements is to be understood to refer to every element in the series. The terms "at least one" and "at least one of" include for example, one, two, three, four, or five or more elements. Slight variations above and below the stated ranges can be used to achieve substantially the same results as values within the ranges. Also, unless indicated otherwise, the disclosure of the ranges is intended as a continuous range including every value between the minimum and maximum values.

## Claims

1. A method of determining the severity of chronic obstructive pulmonary disease in a subject, the method comprising detecting the amount of pro-Pulmonary surfactant-associated protein B (proSP-B), or an effective portion thereof, in a sample from the subject, wherein an increased amount of proSP-B, or the portion thereof, relative to a threshold value, indicates an elevated severity of chronic obstructive pulmonary disease, wherein the sample is a blood sample from a human who does not have a history of heart failure, and wherein a value above 35 pg/ml proSP-B and a value above 65 pg/ml of the portion of proSP-B defined by amino acid positions 285 to 334 of the sequence of SEQ ID NO: 1 indicates increased severity of chronic obstructive pulmonary disease.

2. The method of claim 1, further comprising detecting the amount of at least one additional marker of (i) Brain natriuretic peptide (BNP), (ii) the 76 amino acid N-terminal fragment of Brain natriuretic peptide (NT-pro BNP), (iii) troponin T and (iv) troponin I in the sample, wherein an increased amount of BNP, NT-pro BNP, troponin T and/or troponin I, relative to a threshold value for the additional marker, further indicates a complication of chronic obstructive pulmonary disease.

3. The method of claim 2, wherein the complication of chronic obstructive pulmonary disease is a systemic infection.

4. The method of any one of claims 1 to 3, comprising repeatedly detecting the amount of proSP-B, or an effective portion thereof, in a sample from the subject.

5. The method of any one of claims 1 to 4, wherein detecting the amount of proSP-B, or a portion thereof, comprises contacting the sample with a binding agent, the binding agent being specific for proSP-B, or the portion thereof, and detecting the amount of the binding agent binding to proSP-B.

6. The method of any one of claims 1 to 5, wherein the subject is a human that has previously suffered clinical heart failure and wherein the control sample is one of a blood sample, a plasma sample and a serum sample from the same subject taken at a time where the subject was known (i) to have no chronic obstructive pulmonary disease or (ii) to have no increase in severity of chronic obstructive pulmonary disease.

7. The *in-vitro* use of a binding agent specific for proSP-B, or an effective portion thereof, for assessing the severity of chronic obstructive pulmonary disease in a subject, the use comprising the detection of the amount of proSP-B, or a portion thereof, in a blood sample from a human by means of the binding agent, wherein the human does not have a history of heart failure,
wherein a value above about 35 pg/ml proSP-B and a value above 65 pg/ml of the portion of proSP-B defined by amino acid positions 285 to 334 of the sequence of SEQ ID NO: 1 indicates increased severity of chronic obstructive pulmonary disease.

## Patentansprüche

1. Verfahren zur Bestimmung der Schwere der chronisch obstruktiven Lungenerkrankung in einem Subjekt, wobei das Verfahren umfasst Nachweisen der Menge an pro-pulmonalem Surfactant-assoziierten Protein B (proSP-B) oder eines wirksamen Teils davon in einer Probe des Subjekts, wobei eine erhöhte Menge an proSP-B oder des Teils davon im Verhältnis zu einem Grenzwert eine erhöhte Schwere der chronisch obstruktiven Lungenerkrankung anzeigt, wobei die Probe eine Blutprobe von einem Mensch ist, der keine Vorgeschichte einer Herzinsuffizienz hat, und wobei ein Wert über 35 pg/ml proSP-B und ein Wert über 65 pg/ml des Teils von proSP-B, der durch die Aminosäurepositionen 285 bis 234 der Sequenz von SEQ ID NO: 1 definiert ist, eine erhöhte Schwere der chronisch obstruktiven Lungenerkrankung anzeigt.

2. Verfahren nach Anspruch 1, weiterhin umfassend Nachweisen der Menge von mindestens einem zusätzlichen Marker aus (i) Gehirn-natriuretisches Peptid (BNP), (ii) dem 76 Aminosäure N-terminalen Fragment des Gehirn-natriuretischen Peptids (NT-pro BNP ), (iii) Troponin T und (iv) Troponin I in der Probe, wobei eine erhöhte Menge an BNP, NT-pro BNP, Troponin T und/oder Troponin I im Verhältnis zu einem Grenzwert für den zusätzlichen Marker zusätzlich eine Komplikation der chronisch obstruktiven Lungenerkrankung anzeigt.

3. Verfahren nach Anspruch 2, wobei die Komplikation der chronisch obstruktiven Lungenerkrankung eine systemische Infektion ist.

4. Verfahren nach einem der Ansprüche 1 bis 3, umfassend das mehrmalige Nachweisen der Menge an proSP-B oder eines wirksamen Teils davon in einer Probe des Subjekts.

5. Verfahren nach einem der Ansprüche 1 bis 4, wobei das Nachweisen der Menge an proSP-B oder eines Teils davon Kontaktieren der Probe mit einem Bindemittel umfasst, wobei das Bindemittel spezifisch für proSP-B oder einen Teil davon ist, und Nachweisen der Menge des Bindemittels umfasst.

6. Verfahren nach einem der Ansprüche 1 bis 5, wobei das Subjekt ein Mensch ist, der zuvor an einer klinischen Herzinsuffizienz gelitten hat, und wobei die Kontrollprobe eine aus einer Blutprobe, einer Plasmaprobe und einer Serumprobe von dem gleichen Subjekt ist, entnommen zu einem Zeitpunkt als für das Subjekt bekannt war, dass es (i) keine chronische obstruktive Lungenerkrankung oder (ii) keinen Anstieg in der Schwere der chronisch obstruktiven Lungenerkrankung hat.

7. *In vitro*-Verwendung eines Bindemittels, das spezifisch für proSP-B oder einen wirksamen Teil davon ist, zur Beurteilung der Schwere der chronisch obstruktiven Lungenerkrankung in einem Subjekt, wobei die Verwendung Nachweisen der Menge an proSP-B oder eines Teils davon in einer Blutprobe eines Menschen mit Hilfe des Bindemittels umfasst, wobei der Mensch keine Vorgeschichte einer Herzinsuffizienz hat,
wobei ein Wert über etwa 35 pg/ml proSP-B und ein Wert über 65 pg/ml des Teils von proSP-B, der durch die Aminosäurepositionen 285 bis 234 der Sequenz von SEQ ID NO: 1 definiert ist, eine erhöhte Schwere der chronisch obstruktiven Lungenerkrankung anzeigt.

## Revendications

1. Procédé de détermination de la gravité d'une bronchopneumopathie chronique obstructive chez un sujet, le procédé comprenant la détection de la quantité de pro-protéine B associée au surfactant pulmonaire (proSP-B), ou d'une portion efficace de celle-ci, dans un échantillon du sujet, dans lequel une quantité augmentée de proSP-B, ou de la portion de celle-ci, par rapport à une valeur seuil, indique une gravité élevée d'une bronchopneumopathie chronique obstructive, dans lequel l'échantillon est un échantillon de sang d'un humain qui n'a pas d'antécédents d'insuffisance cardiaque, et dans lequel une valeur supérieure à 35 pg / ml de proSP-B et une valeur supérieure à 65 pg/ ml de la portion de proSP-B définie par les positions 285 à 334 d'acides aminés de la séquence de SEQ ID N° 1 indiquent une gravité augmentée d'une bronchopneumopathie chronique obstructive.

2. Procédé selon la revendication 1, comprenant en outre la détection de la quantité d'au moins un marqueur supplémentaire (i) du peptide natriurétique cérébral (BNP), (ii) du fragment d'extrémité N de l'acide aminé 76 du peptide natriurétique cérébral (NT-pro BNP), (iii) de la troponine T et (iv) de la troponine I dans l'échantillon, dans lequel une quantité augmentée de BNP, NT-pro BNP, troponine T et / ou troponine I, par rapport à une valeur seuil pour le marqueur supplémentaire, indique en outre une complication d'une bronchopneumopathie chronique obstructive.

3. Procédé selon la revendication 2, dans lequel la complication d'une bronchopneumopathie chronique obstructive est une infection généralisée.

4. Procédé selon l'une quelconque des revendications 1 à 3, comprenant la détection répétée de la quantité de proSP-B, ou d'une portion efficace de celle-ci, dans un échantillon du sujet.

5. Procédé selon l'une quelconque des revendications 1 à 4, dans lequel la détection de la quantité de proSP-B, ou d'une portion de celle-ci, comprend la mise en contact de l'échantillon avec un agent de liaison, l'agent de liaison étant spécifique pour proSP-B, ou la portion de celle-ci, et la détection de la quantité de l'agent de liaison se liant à proSP-B.

6. Procédé selon l'une quelconque des revendications 1 à 5, dans lequel le sujet est un humain qui a précédemment souffert d'une insuffisance cardiaque clinique et dans lequel l'échantillon témoin est un parmi un échantillon de sang, un échantillon de plasma et un échantillon de sérum du même sujet pris à un moment où il était connu que le sujet (i) n'avait pas de bronchopneumopathie chronique obstructive ou (ii) n'avait pas d'augmentation de la gravité d'une bronchopneumopathie chronique obstructive.

7. Utilisation *in-vitro* d'un agent de liaison spécifique à proSP-B, ou à une portion efficace de celle-ci, pour évaluer la gravité d'une bronchopneumopathie chronique obstructive chez un sujet, l'utilisation comprenant la détection de la quantité de proSP-B, ou d'une portion de celle-ci, dans un échantillon de sang d'un humain au moyen de l'agent de liaison, dans laquelle l'humain n'a pas d'antécédents d'insuffisance cardiaque,
dans laquelle une valeur supérieure à 35 pg / ml de proSP-B et une valeur supérieure à 65 pg / ml de la portion de proSP-B définie par les positions 285 à 334 d'acides aminés de la séquence de SEQ ID N° 1 indiquent une gravité augmentée d'une bronchopneumopathie chronique obstructive.
